# EUROPÄISCHE PATENTSCHRIFT

(11) **EP 3 448 329 B1**
(45) Veröffentlichungstag und Bekanntmachung des Hinweises auf die Patenterteilung: **16.03.2022**
(21) Anmeldenummer: 17723301.2
(22) Anmeldetag: 27.04.2017
(51) Int. Cl.: A61F 5/01

(54) **ORTHESENGELENK, BAUKASTENSYSTEM ZUR BILDUNG EINES ORTHESENGELENKES UND VERWENDUNG DESSEN**
ARTICULATION FOR AN ORTHOSIS, ORTHOSIS KIT AND USE
ARTICULATION POUR UNE ORTHESE, KIT D'ASSEMBLAGE ET UTILISATION

(30) Priorität: 27.04.2016 DE 102016107779
(43) Veröffentlichungstag der Anmeldung: 06.03.2019
(73) Patentinhaber: Böckh, Thomas, 85541 Kirchheim b. München (DE)
(72) Erfinder: BÖCKH, Thomas, 85541 Kirchheim b. München (DE); MOMBOISSE, Michel, 81669 München (DE)
(74) Vertreter: Grape & Schwarzensteiner
(86) Internationale Anmeldenummer: PCT/EP2017/060136
(87) Internationale Veröffentlichungsnummer: WO 2017/186885

(56) Entgegenhaltungen:
- DE-A1-102013 011 382
- DE-U1-202011 004 130
- US-A- 5 399 149

## Beschreibung

Die Erfindung betrifft ein Orthesengelenk mit einem Oberteil und einem Fußbügel, die miteinander über ein Drehgelenk mit einer Drehachse zueinander relativ verschwenkbar verbunden sind, ein Baukastensystem zur Bildung eines Orthesengelenkes und dessen Verwendung.

Derartige Orthesen sind allgemein bekannt. Orthesen sind medizinische Hilfsmittel, die zur Stabilisierung, Entlastung, Ruhigstellung, Führung oder Korrektur von Gliedmaßen oder des Rumpfes von Menschen eingesetzt werden. Stützstrukturen in Form von Schalen oder Schienen werden zu diesem Zweck an den Gliedmaßen befestigt. Im Bereich von Gelenken der Menschen sind solche Stützstrukturen ebenfalls mit Gelenken versehen, um eine bestimmte Beweglichkeit zu erreichen. In Abhängigkeit von medizinischen und/oder therapeutischen Erfordernissen, kann die Bewegung mit solchen Stützstrukturen richtungs- und/oder betragsmäßig eingeschränkt, durch einen langsam oder (unterschiedlich) schnell ansteigenden Widerstand individuell verzögert oder aufgehoben oder durch eine Rückstellkraft zur Rückkehr in eine Ausgangsstellung unterstützt werden.

Die US-A-4,771,768 betrifft eine Knöchelorthese zur Behandlung von Knöchelbrüchen mit einem Oberteil in Form einer Unterschenkelschiene und einem daran gelenkig befestigten Fußteil. An dem Oberteil ist ein Grundkörper angeordnet, über den mittels zweier Einstellschrauben und zweier von den Einstellschrauben beaufschlagbaren Metallstifte die Beweglichkeit der Knöchelorthese und der maximale Bewegungswinkel eingestellt werden sollen.

In der US 5 399 149 A ist ein Kniegelenk von selektiv begrenzbarer Bewegung mit in der Gelenkverbindung integral angeordneten federelastischen Funktionselementen beschrieben.

Die DE 10 2010 014 334 A1 bzw. DE 20 2011 004 130 U1 beschreiben ein Orthesengelenk mit einem Grundkörper, der eine Aufnahme für eine proximale Komponente bzw. Orthesenschiene und Funktionsmittel zum Bilden eines federnden Dorsalanschlags und eines federnden Plantaranschlags aufweist. Die von dem Grundkörper gesonderte Orthesenschiene ist von der Aufnahme in dem Grundkörper aufgenommen und über zwei Schrauben an dem Grundkörper festgelegt. Die Funktionsmittel sind als Tellerfederanordnung ausgebildet und von Aufnahmeelementen aufgenommen, die durch Einschrauben in Gewinde des Grundkörpers an dem Grundkörper befestigbar sind. Um die Aufnahmeelemente herum sind als gesonderte Bauteile ausgebildete Verkleidungsteile angeordnet. Auf diese Weise soll ein möglichst glattwandiger und kompakter Aufbau des Orthesengelenkes ermöglicht werden. Über Kanäle in dem Grundkörper wirken die Funktionsmittel auf eine/n schwenkbar an dem Grundkörper angeordnete/n distale Komponente bzw. Fußbügel. Die Vorspannung der Tellerfederanordnung kann über ein Stellelement, das in jeden Kanal eingeschraubt wird, verändert werden. Nicht zuletzt aufgrund der ausgesprochen vielen einzelnen und voneinander gesonderten Bauteile gestaltet sich dieses Orthesengelenk in seiner Bauweise als aufwendig. Ein modularer Aufbau des Orthesengelenkes ist durch eine strikt vorgegebene Ausbildung von Grundkörper, Orthesenschiene und Fußbügel sowie deren gegenseitige Anordnung zueinander nicht möglich.

Die DE 10 2013 011 382 A1, welche den nächstliegenden Stand der Technik darstellt, zeigt schließlich ebenfalls ein Orthesengelenk von entsprechend nachteiliger Konstruktion, d.h. also wiederum mit einem Grundkörper, der eine Aufnahme für eine proximale Komponente und eine Lagerstelle für eine schwenkbar an dem Grundkörper angeordnete distale Komponente sowie auf die distale Komponente einwirkende Anschläge oder Federelemente aufweist. In oder an dem Aufnahmeelement ist ein mit der distalen Komponente wechselwirkendes Funktionselement angeordnet.

Bei sämtlichen dieser bekannten Orthesengelenken hat sich allerdings in der Praxis als nachteilig erwiesen, dass deren konstruktive Ausgestaltung lediglich auf den jeweiligen, d.h. einen ganz bestimmten und/oder sehr begrenzten, Einsatzzweck abgestimmt sind. Eine Individualisierung des Orthesengelenkes und Anpassung bzw. Abstimmung an die medizinischen und/oder therapeutischen Erfordernisse des jeweiligen Patienten und dessen Krankheitsbild und/oder Therapiefortschritt ist - nicht zuletzt aufgrund der vielen einzelnen, zum Teil sehr unterschiedlichen - Bauteilen - nur mit erheblichem Aufwand und ausgesprochen hohen Betriebs-, Fertigungs- Vorhaltungs- und Lagerkosten verbunden. Eine nachträgliche Anpassung bzw. Abstimmung des Orthesengelenkes an veränderte medizinische und/oder therapeutische Erfordernisse des jeweiligen Patienten und dessen Krankheitsbild und/oder Therapiefortschritt ist zudem vielfach ausgeschlossen. Dies umso mehr, wenn die zwischenzeitlichen Veränderungen und/oder Therapiefortschritte gravierend sind. Gerade in solchen Fällen müssen die Patienten aus Kostengründen auf eine nachträgliche Anpassung bzw. Abstimmung des Orthesengelenkes verzichten, wodurch wiederum die Therapiefortschritte und -erfolge wesentlich beeinträchtigt sind/werden.

Der Erfindung liegt daher die Aufgabe zugrunde, ein Orthesengelenk zur Verfügung zu stellen, mit welcher sich die obigen Nachteile verhindern lassen, welches mithin konstruktiv besonders einfach, damit einhergehend leicht, schnell und besonders kostengünstig an die körperlichen und gesundheitlichen Gegebenheiten des Menschen individuell anpassbar ist, dabei zugleich eine ganz exakte Feineinstellung auf diese persönlichen körperlichen Gegebenheiten des Menschen ermöglicht und damit einhergehend zu einer erheblichen Reduzierung des Kostenaufwandes insgesamt führt, sowie eine vorteilhafte Verwendung dieses Orthesengelenkes bereitzustellen.

Diese Aufgabe wird in vorrichtungstechnischer Hinsicht durch die Merkmale des Anspruchs 1 gelöst.

Durch die Ausgestaltung des erfindungsgemäßen Orthesengelenkes mit einem Oberteil und einem Fußbügel, die miteinander über ein Drehgelenk mit einer Drehachse zueinander relativ verschwenkbar verbunden sind, wobei der Fußbügel zu beiden Seiten des Drehgelenkes durch ein in/von einem Aufnahmeelement aufgenommenes, federelastisches Funktionselement dorsal und/oder plantar beaufschlagbar ist, wobei das Aufnahmeelement über einen sich von einer dorsalen Seite zu einer plantaren Seite, und umgekehrt, quer zu einer Längsachse des Oberteiles erstreckenden Befestigungsabschnitt des Aufnahmeelementes an dem Oberteil lösbar befestigbar ist und wenigstens einen, insbesondere zwei, an dem Befestigungsabschnitt angeordnete/n Aufnahmeabschnitt/e zur Aufnahme des/der Funktionselemente/s seitlich des Oberteiles umfasst, ist eine besonders einfache Bauweise des erfindungsgemäßen Orthesengelenkes insgesamt erreicht. Darüber hinaus hat das erfindungsgemäße Orthesengelenk den wesentlichen Vorteil, leicht, schnell und besonders kostengünstig an die körperlichen und gesundheitlichen Gegebenheiten, die körperliche Konstitution, das Alter, das Krankheitsbild und/oder den Therapiefortschritt etc., des Menschen oder Patienten individuell angepasst werden zu können. Zudem ist eine ausgesprochen exakte Feineinstellung möglich. Nicht zuletzt hieraus resultierend ergibt sich eine ausgesprochen leichte Handhabung des erfindungsgemäßen Orthesengelenkes insgesamt bei der Anpassung und Feineinstellung des Orthesengelenkes nach der Erfindung an den individuellen Körper des Menschen. Damit einhergehend lassen sich zugleich die Betriebs-, Fertigungs- Vorhaltungs- und Lagerkosten erheblich vermindern. Ganz wesentliche Vorteile, die sich mit dem erfindungsgemäßen Orthesengelenk ergeben, sind einerseits eine erhebliche Reduzierung der Anzahl der Bauteile und andererseits eine Standardisierung bzw. Normierung der noch verwendeten (wenigen) Bauteile um jeweils ein größtmögliches Maß. Mit dem erfindungsgemäßen Orthesengelenk lässt sich ein Baukastensystem erhalten, das eine beliebige und vollkommen individuelle Anpassung und Abstimmung des Orthesengelenkes auf die körperlichen und gesundheitlichen Gegebenheiten, die körperliche Konstitution, das Alter, das Krankheitsbild und/oder den Therapiefortschritt etc., des Menschen oder Patienten ermöglicht, wobei sämtliche medizinischen und/oder therapeutischen Erfordernisse vollumfänglich berücksichtigt werden (können).

Weitere vorteilhafte Einzelheiten der erfindungsgemäßen Orthese sind in den Ansprüchen 2 bis 24 beschrieben.

Erfindungsgemäß ist vorgesehen, dass das Aufnahmeelement nach Anspruch 2 vorteilhafterweise einen sich von der dorsalen Seite zur plantaren Seite, und umgekehrt, im Wesentlichen senkrecht oder senkrecht oder in einem Winkel schräg zur Längsachse des Oberteiles erstreckenden Befestigungsabschnitt aufweist.

Von ganz besonderer Bedeutung für eine einfache, schnelle und vor allem genaue (Fein-)Einstellung und Anpassung des Orthesengelenkes an die Körperverhältnisse des Menschen ist die konstruktive Ausgestaltung nach Anspruch 3. Das Aufnahmeelement ist demnach mit dem Befestigungsabschnitt und dem wenigstens einen, insbesondere den zwei, an dem Befestigungsabschnitt angeordneten Aufnahmeabschnitt/en einstückig ausgebildet. Die Montage und/oder Demontage bzw. Bestückung des Orthesengelenkes gestaltet sich mit sehr wenigen Handgriffen ausgesprochen einfach und schnell.

Besonders vorteilhaft für eine Standardisierung bzw. Normierung der einzelnen Bauteile sind die Merkmale des Anspruchs 4, wonach das Oberteil, an dem das Aufnahmeelement lösbar befestigt ist, einstückig bzw. einteilig bzw. integral ausgebildet ist, wodurch sich die Anzahl von Bauteilen zusätzlich verringern lässt. Mithin besteht das Oberteil aus einem oberen Ende und dem unteren Ende und ist einstückig bzw. einteilig bzw. integral ausgebildet. Die eigentliche proximale Komponente bzw. Orthesenschiene und ein Teil des Drehgelenkes sind daher nicht - wie im Stand der Technik - als gesonderte, voneinander unabhängige und erst zusammenzufügende und miteinander zu verbindende bzw. zu befestigende Bauteile ausgebildet. Auf diese Weise lassen sich gegenüber dem Stand der Technik die Anzahl von einzelnen Bauteilen erheblich vermindern, damit der Aufwand in Herstellung und Kosten wesentlich reduzieren und zugleich die Stabilität der erfindungsgemäßen Orthesengelenkes sowie damit einhergehend das äußere Erscheinungsbild bzw. Design verbessern.

Nach Anspruch 5 ist das Aufnahmeelement an dem Oberteil form- und/oder kraftschlüssig befestigbar.

Von besonderem Interesse sind die Maßnahmen des Anspruchs 6. Danach ist das Oberteil mit einer sich quer, insbesondere im Wesentlichen senkrecht oder senkrechte oder in einem Winkel schräg, zur Längsachse des Oberteiles erstreckenden Nut oder dergleichen Ausnehmung versehen, in welche das Aufnahmeelement, insbesondere der Befestigungsabschnitt des Aufnahmeelementes, einsetzbar ist. Durch Aufnahme des Aufnahmeelementes in/von der Nut oder dergleichen Ausnehmung werden die im Betrieb auftretenden, üblicherweise beträchtlichen Kräfte und Momente unmittelbar in Oberteil und/oder Fußbügel eingeleitet bzw. übertragen. Eine Beschädigung oder Zerstörung von Verbindungselementen, wie Schrauben oder dergleichen, durch Belastungen aufgrund der Kräfte und/oder Momente oder sogar eine Beschädigung des erfindungsgemäßen Orthesengelenkes insgesamt ist ausgeschlossen. Die umso mehr, wenn das Aufnahmeelement von der Nut oder dergleichen Ausnehmung mit geringem oder bevorzugt ohne Spiel, aufgenommen ist.

Vorteilhafterweise ist das Aufnahmeelement nach Anspruch 7 an dem Oberteil über eine Schraub-, Rast-, Schnapp- oder dergleichen -verbindung befestigbar.

Darüber hinaus ist/sind der wenigstens eine, insbesondere die zwei, an dem Befestigungsabschnitt angeformte/n Aufnahmeabschnitt/e des Aufnahmeelementes nach Anspruch 8 parallel und/oder in einem Winkel schräg zur Längsachse des Oberteiles und/oder symmetrisch beidseits der Längsachse des Oberteiles und zueinander angeordnet.

Nach Anspruch 9 liegt/liegen der wenigstens eine, insbesondere die zwei, an dem Befestigungsabschnitt angeformte/n Aufnahmeabschnitt/e des Aufnahmeelementes an der/die dorsale/n bzw. vordere/n Seite bzw. Seitenkante und/oder der/die plantare/n bzw. hintere/n Seite bzw. Seitenkante des Oberteiles seitlich an oder berührt/berühren diese. Auf diese Weise lässt sich ein zusätzlicher Formschluss erreichen, um auftretende Kräfte und/oder Momente unmittelbar auf Oberteil zu übertragen.

Neben einer einfachen, schnellen und individuellen Handhabung sowie Anpassung des Orthesengelenkes nach der Erfindung bei der medizinischen und/oder therapeutischen Anwendung und einer zusätzlichen Standardisierung bzw. Normierung der einzelnen Bauteile sind die Merkmale des Anspruchs 10, wonach der wenigstens eine, insbesondere die zwei, an dem Befestigungsabschnitt angeformte/n Aufnahmeabschnitt/e im Wesentlichen gekapselt ausgebildet ist/sind, weiter von ausgesprochen großer Bedeutung. So lassen sich in dem gekapselten Aufnahmeelement beliebig ausgestaltete Funktionselemente einbringen. Darüber hinaus lassen sich zugleich Vorteile hinsichtlich einer fehlenden Verschmutzung des/der Funktionselemente/s und somit einer unaufwendigen Sauberhaltung des erfindungsgemäßen Orthesengelenkes und Hygiene erzielen.

In diesem Zusammenhang ist/sind der wenigstens eine, insbesondere die zwei, an dem Befestigungsabschnitt angeformte/n Aufnahmeabschnitt/e nach Anspruch 11 jeweils mit einer länglichen Ausnehmung oder Bohrung zur Aufnahme des Funktionselementes versehen.

Darüber hinaus liegt es im Rahmen der Erfindung, dass der wenigstens eine, insbesondere die zwei, an dem Befestigungsabschnitt angeformte/n Aufnahmeabschnitt/e nach Anspruch 12 eine, insbesondere mittels Verschraubung, verschließbare Öffnung zur Einbringung des Funktionselementes umfasst/umfassen.

Der wenigstens eine, insbesondere die zwei, an dem Befestigungsabschnitt angeformte/n Aufnahmeabschnitt/e umfasst/umfassen nach Anspruch 13 in vorteilhafter Weise ein in dem Aufnahmeabschnitt axial verschiebbares und von dem Funktionselement beaufschlagtes Betätigungselement zur dorsalen und/oder plantaren Beaufschlagung des Fußbügels.

Weiterhin ist erfindungsgemäß vorgesehen, dass der wenigstens eine, insbesondere die zwei, an dem Befestigungsabschnitt angeformte/n Aufnahmeabschnitt/e nach Anspruch 14 ein von dem Funktionselement beaufschlagtes und sich teilweise durch eine Durchgangsbohrung oder dergleichen Durchgang erstreckendes Betätigungselement zur dorsalen und/oder plantaren Beaufschlagung des Fußbügels umfasst/umfassen.

Zweckmäßigerweise ist das Betätigungselement zur dorsalen und/oder plantaren Beaufschlagung des Fußbügels nach Anspruch 15 durch ein Funktionselement beaufschlagt und mit einem sich durch die Durchgangsbohrung oder dergleichen Durchgang erstreckenden Stift oder Bolzen gebildet.

Entsprechend den Merkmalen des Anspruchs 16 ist/sind der wenigstens eine, insbesondere die zwei, an dem Befestigungsabschnitt angeformte/n Aufnahmeabschnitt/e im Wesentlichen freiliegend ausgebildet.

Dabei ist/sind der wenigstens eine, insbesondere die zwei, an dem Befestigungsabschnitt angeformte/n Aufnahmeabschnitt/e nach Anspruch 17 vorzugsweise mit Schrauben, Vorsprüngen oder nutförmigen Ausnehmungen zur Aufnahme und Halterung des Funktionselementes versehen.

Die Merkmale des Anspruchs 18, dass dem Oberteil ein Keilelement, über welches die Bewegungsfreiheit des Orthesengelenkes mittels wenigstens einer, vorzugsweise zwei, Keilfläche/n einstellbar ist, zugeordnet ist, sorgen für eine vereinfachte Einstellung der Bewegungsfreiheit des erfindungsgemäßen Orthesengelenkes und damit Anpassung sowie Feineinstellung an die körperlichen Gegebenheiten des Menschen.

In diesem Zusammenhang erweisen sich die Merkmale des Anspruchs 19 als besonders zweckmäßig für eine einfache wie gleichermaßen schnelle Handhabung des erfindungsgemäßen Orthesengelenkes. Danach ist das Oberteil mit einer sich im Wesentlichen senkrecht zur Längsachse des Oberteiles erstreckenden Nut oder dergleichen Ausnehmung versehen, in welche das Keilelement einsetzbar und lösbar befestigt ist. Vorzugsweise ist das Keilelement in der Praxis ein einem Trägerelement befestigbar und mittelbar von der Nut oder dergleichen Ausnehmung aufgenommen. Ohne im Einzelnen dargestellt zu sein, kann das Keilelement ohne weiteres auch unmittelbar, d.h. ohne ein solches Trägerelement, in die Nut oder dergleichen Ausnehmung eingebracht werden/sein.

Von besonderem Interesse sind weiterhin die Maßnahmen, wonach dem Fußbügel ein Stellelement, über welches die Funktionsstellung des Orthesengelenkes mittels einer der wenigstens einen Keilfläche zugewandten und mit der wenigstens eine Keilfläche zusammenwirkenden Anschlagfläche einstellbar ist, zugeordnet ist. Die Einstellung der Funktionsstellung des erfindungsgemäßen Orthesengelenkes und damit Anpassung sowie Feineinstellung an die körperlichen Gegebenheiten des Menschen gestalten sich ausgesprochen einfach und schnell.

Dadurch, dass nämlich der Fußbügel mit dem Stellelement lösbar verbunden ist, lassen sich die Handhabung des Orthesengelenkes nach der Erfindung bei der medizinischen und/oder therapeutischen Anwendung noch zusätzlich verbessern und die Standardisierung bzw. Normierung der einzelnen Bauteile weiter ausbilden.

Weiterhin liegt es im Rahmen der Erfindung, den Fußbügel und das Stellelement mit einer Rast- oder Zahnverbindung zur stufenverstellbaren Einstellung der Funktionsstellung des Orthesengelenkes miteinander lösbar zu verbinden.

Dabei ist die Rast- oder Zahnverbindung zwischen Fußbügel und Stellelement bevorzugt in einer im Wesentlichen halbkreis- oder teilhalbkreisförmigen Anordnung um das Drehgelenk herum angeordnet.

Die Rast- oder Zahnverbindung weist zwischen Fußbügel und Stellelement eine gleichmäßige Teilung auf, durch welche die Funktionsstellung des Orthesengelenkes in regelmäßigen Stufen einstellbar ist.

Von ganz besonderem Vorteil für eine vereinfachte Herstellung einerseits und eine variable Anpassung und vielseitige Verwendung des erfindungsgemäßen Orthesengelenkes ist der Fußbügel mit zwei Rast- oder Zahnverbindungen ausgestattet, deren Teilung jeweils miteinander übereinstimmt und relativ zueinander versetzt ist, und dass das Stellelement eine Rast- oder Zahnverbindung aufweist, deren Tiefe im Wesentlichen an die Tiefe einer der zwei Rast- oder Zahnverbindungen des Fußbügels angepasst ist. Hierdurch wird eine sehr feine Stufenverstellbarkeit des Stellelementes und damit der Einstellung der Funktionsstellung des Orthesengelenkes ermöglicht, ohne eine zu feine Rast- und Zahnverbindung mit damit einhergehend erhöhten Kosten herstellen zu müssen. Durch den Versatz ist es möglich, beispielsweise bei Verbindung des Stellelementes mit dem Fußbügel von dessen Oberseite her durch die eine Rast- und Zahnverbindung eine Teilung von 0°, 10°, 20°, 30°, 40°, ..., und bei Verbindung des Stellelementes mit dem Fußbügel von dessen Unterseite her durch die andere Rast- und Zahnverbindung eine Teilung von 5°, 15°, 25°, 35°, 45°, ..., zur Verfügung zu haben.

Zusätzlich ist erfindungsgemäß vorgesehen, dass die zwei Rast- oder Zahnverbindungen des Fußbügels und die eine Rast- oder Zahnverbindung des Stellelementes jeweils eine Tiefe entsprechend der halben Dicke des Fußbügels aufweisen.

Des Weiteren sind eine der zwei Rast- oder Zahnverbindungen des Fußbügels und die eine Rast- oder Zahnverbindung des Stellelementes von der Oberseite des Fußbügels und die andere der zwei Rast- oder Zahnverbindungen des Fußbügels und die eine Rast- oder Zahnverbindung des Stellelementes von der Unterseite des Fußbügels in Wirkverbindung bringbar.

In vorteilhafter Weise ist der Fußbügel beidseitig mit einem ringförmigen, um die Ausnehmung wenigstens teilweise, insbesondere vollständig, umlaufenden Vorsprung oder dergleichen Wulst versehen ist, der mit einer Innenwandung einer kreisförmigen Ausnehmung des Stellelementes zur Anlage bringbar.

Darüber hinaus weist die kreisförmige Ausnehmung des Stellelementes einen Durchmesser auf, welcher einem Durchmesser einer Ausnehmung oder einem Außendurchmesser eines Achsschenkels oder dergleichen Lagerbolzens des Drehgelenkes zu dessen, insbesondere spielfreien, Aufnahme zuzüglich der radialen Breite bzw. Dicke des ringförmigen Vorsprunges des Fußbügels entspricht.

Weiterhin liegt es im Rahmen der Erfindung, dass der Fußbügel zu beiden Seiten des Drehgelenkes nach Anspruch 20 einen dorsalen und einen plantaren Vorsprung oder dergleichen Auskragung umfasst, der/die durch das in/von einem Aufnahmeelement aufgenommene, federelastische Funktionselement beaufschlagbar ist.

In ganz vorteilhafter Weise ist dem Fußbügel ein Stellelement, über welches die Funktionsstellung des Orthesengelenkes mittels einer der wenigstens einen Keilfläche zugewandten und mit der wenigstens einen Keilfläche zusammenwirkenden Anschlagfläche einstellbar ist, zugeordnet, wobei der dorsale und der plantare Vorsprung oder dergleichen Auskragung des Fußbügels zu beiden Seiten des Drehgelenkes nach Anspruch 21 einstückig an dem Stellelement angeformt ist. Auf diese Weise lässt sich zum einen die Anzahl der Bauteile vermindern. Zum anderen ist eine zusätzliche Standardisierung bzw. Normierung der Bauteile ermöglicht. So ist es denkbar, dass das erfindungsgemäße Orthesengelenk zunächst zusammen mit einem oder mehreren verschiedenen federelastischen Funktionselementen zur Anwendung kommt. Nach einem erkennbaren Therapiefortschritt gelangt sodann ein Orthesengelenk ohne federelastisches Funktionselement zur Anwendung. In diesem Fall ist es nicht erforderlich, das gesamte Orthesengelenk auszutauschen. Vielmehr muss lediglich das Stellelement mit einem dorsalen und einem plantaren Vorsprung oder dergleichen Auskragung zu beiden Seiten des Drehgelenkes durch ein entsprechendes Stellelement, das solche Vorsprünge oder Auskragungen nicht aufweist zu ersetzen.

Darüber hinaus ist/sind das/die Funktionselement/e entsprechend den Maßnahmen nach Anspruch 22 bevorzugterweise als mindestens eine Feder, insbesondere Druckfeder, vorzugsweise Tellerfeder und/oder Schraubenfeder und/oder Kunststoffelement, oder insbesondere Zugfeder, vorzugsweise Schraubenfeder und/oder Ringfeder aus federelastischem Material, und/oder eine Gasdruckfeder und/oder eine Gaszugfeder, und/oder einen Dämpfer und/oder einen Motor, insbesondere Schrittmotor, und/oder einer magnetischen Einrichtung, insbesondere einen Magneten, und/oder einer Kombination daraus und/oder einem daraus gebildeten Paket, ausgebildet.

Diese Aufgabe wird weiter in vorrichtungstechnischer Hinsicht durch die Merkmale des Anspruchs 23 gelöst.

Demnach lässt sich mit dem Orthesengelenk nach der Erfindung ein Baukastensystem auf Basis von wenigen, zudem standardisierten bzw. normierten Bauteilen erhalten, die sich gegeneinander beliebig austauschen und/oder ersetzen lassen. Damit ist eine beliebige und vollkommen individuelle Anpassung und Abstimmung des Orthesengelenkes auf die körperlichen und gesundheitlichen Gegebenheiten, die körperliche Konstitution, das Alter, das Krankheitsbild und/oder den Therapiefortschritt etc., des Menschen oder Patienten ermöglich, unter vollumfänglicher Berücksichtigung und Einbeziehung sämtlicher medizinischer und/oder therapeutischer Erfordernisse und Maßnahmen.

Diese Aufgabe wird schließlich noch in verwendungsmäßiger Hinsicht durch die Merkmale des Anspruchs 24 gelöst.

Danach liegt es im Rahmen der Erfindung, das erfindungsgemäße Orthesengelenk an/mit Orthesen unterer Gliedmaßen, insbesondere Fuß-Orthesen ((Foot-Orthoses) (FO)), Knöchel-Fuß-Orthesen (Ankle-Foot-Orthoses) (AFO)), Knie-Knöchel-Orthesen ((Knee-Ankle-Orthoses) (KAO)), Knie-Knöchel-Fuß-Orthesen (Knee-Ankle-Foot-Orthoses) (KAFO)), Hüfte-Knie-Knöchel-Orthesen ((Hip-Knee-Ankle-Orthoses) (HKAO)), Hüfte-Knie-Orthesen ((Hip-Knee-Orthoses) (HKO)), Hüfte-Knie-Knöchel-Fuß-Orthesen ((Hip-Knee-Ankle-Foot-Orthoses) (HKAFO)), Reziproke-Geh-Orthesen (Reciprocating-Gait-Orthoses) (RGO)), Rumpf-Orthesen (Torso-Orthoses), Ellbogen-Orthesen (Elbow-Orthoses), Ellbogen-Schulter-Orthesen (Elbow-Shoulder-Orthoses), Rumpf-Schulter-Orthesen (Torso-Shoulder-Orthoses), Rumpf-Schulter-Ellbogen-Orthese (Torso-Shoulder-Elbow-Orthoses), Schulter-Orthesen (Shoulder-Orthoses), Prothese mit Oberschenkelhülsen (Prostheses with above knee socket) oder Prothesen mit Beckenkorb (Prostheses with pelvic socket), zu verwenden.

Weitere Merkmale, Vorteile und Einzelheiten der Erfindung ergeben sich aus der nachfolgenden Beschreibung von einigen bevorzugten Ausführungsformen der Erfindung sowie anhand der Zeichnungen. Hierbei zeigen:
Fig. 1a bis 1e eine perspektivische Ansicht, eine Draufsicht, eine Seitenansicht, eine Unteransicht und eine Vorderansicht und eine Oberansicht einer Ausführungsform einer erfindungsgemäß ausgebildeten Gelenkorthese,
Fig. 2a bis 2e eine perspektivische Ansicht, eine Draufsicht, eine linke und eine rechte Seitenansicht und eine Unteransicht einer Ausführungsform einer erfindungsgemäß ausgebildeten Oberteiles entsprechend den Fig. 1a bis 1e,
Fig. 3a und 3g eine perspektivische Ansicht, eine Draufsicht, eine linke und eine rechte Seitenansicht, eine Unteransicht und eine Vorderansicht einer Ausführungsform eines erfindungsgemäß ausgebildeten Fußbügels entsprechend den Fig. 1a bis 1e sowie eine Schnittansicht gemäß Ausschnitt IIIG in der Fig. 3d, in vergrößerter Darstellung,
Fig. 4a bis 4d teilweise abgebrochene Schnittansichten durch verschiedene Ausführungsformen erfindungsgemäß ausgebildeter Orthesengelenke im Bereich des Drehgelenkes entsprechend der Fig. 3a bis 3g, in vergrößerter Darstellung,
Fig. 5a bis 5e perspektivische Vorder- und Rückansicht, eine auseinandergezogene Ansicht, eine Seitenansicht und eine Vorderansicht eines erfindungsgemäß ausgebildeten Keilelementes,
Fig. 6a bis 6g verschiedene perspektivische Vorderansichten, eine Draufsicht, eine linke und eine rechte Seitenansicht, eine Vorder- bzw. Rückansicht und eine Unteransicht eines erfindungsgemäß ausgebildeten Stellelementes,
Fig. 7a bis 7g teilweise abgebrochene Schnittansichten durch einen erfindungsgemäß ausgebildeten Fußbügel entsprechend der Fig. 3a bis 3g zusammen mit einem erfindungsgemäßen Stellelement entsprechend der Fig. 5a bis 5g in einer Stellung senkrecht zur Längsachse des Fußbügels und mit dazu unterschiedlichen Stellungen,
Fig. 8a bis 8e eine perspektivische Ansicht, eine linke Seitenansicht, eine Draufsicht, eine rechte Seitenansicht und eine Unteransicht einer weiteren Ausführungsform einer erfindungsgemäß ausgebildeten Gelenkorthese,
Fig. 9a bis 9f eine perspektivische Ansicht, eine Draufsicht, eine linke und eine rechte Seitenansicht, eine Vorder- bzw. Rückansicht und eine Unteransicht einer weiteren Ausführungsform eines erfindungsgemäß ausgebildeten Stellelementes entsprechend den Fig. 8a bis 8e,
Fig. 10 eine teilweise abgebrochene Schnittansicht durch die Ausführungsform des erfindungsgemäß ausgebildeten Orthesengelenkes im Bereich des Drehgelenkes entsprechend den Fig. 8a bis 8e, in vergrößerter Darstellung,
Fig. 11a bis 11e eine perspektivische Ansicht, eine linke Seitenansicht, eine Draufsicht, eine rechte Seitenansicht und eine Unteransicht einer noch weiteren Ausführungsform einer erfindungsgemäß ausgebildeten Gelenkorthese,
Fig. 12a bis 12f eine perspektivische Ansicht, eine Draufsicht, eine linke und eine rechte Seitenansicht, eine Vorder- bzw. Rückansicht und eine Unteransicht einer weiteren Ausführungsform eines erfindungsgemäß ausgebildeten Stellelementes entsprechend den Fig. 11a bis 11e, und
Fig. 13 eine teilweise abgebrochene Schnittansicht durch die Ausführungsform des erfindungsgemäß ausgebildeten Orthesengelenkes im Bereich des Drehgelenkes entsprechend den Fig. 11a bis 11e, in vergrößerter Darstellung.

Bei der nachfolgenden Beschreibung von verschiedenen Ausführungsformen eines erfindungsgemäß ausgebildeten Orthesengelenkes 10 sind einander entsprechende, gleiche Bauteile jeweils mit identischen Bezugsziffern versehen.

Das Orthesengelenk 10 nach der Erfindung ist zur Fortbewegung eines Menschen, der an Cerebarparese, Infantiler Cerebarparese (ICP) oder dergleichen Erkrankung leidet, vorgesehen. Insbesondere eignet sich das erfindungsgemäße Orthesengelenk 10 zur Verwendung an/mit Orthesen unterer Gliedmaßen, insbesondere Fuß-Orthesen ((Foot-Orthoses) (FO)), Knöchel-Fuß-Orthesen (Ankle-Foot-Orthoses) (AFO)), Knie-Knöchel-Orthesen ((Knee-Ankle-Orthoses) (KAO)), Knie-Knöchel-Fuß-Orthesen (Knee-Ankle-Foot-Orthoses) (KAFO)), Hüfte-Knie-Knöchel-Orthesen ((Hip-Knee-Ankle-Orthoses) (HKAO)), Hüfte-Knie-Orthesen ((Hip-Knee-Orthoses) (HKO)), Hüfte-Knie-Knöchel-Fuß-Orthesen ((Hip-Knee-Ankle-Foot-Orthoses) (HKAFO)), Reziproke-Geh-Orthesen (Reciprocating-Gait-Orthoses) (RGO)), Rumpf-Orthesen (Torso-Orthoses), Ellbogen-Orthesen (Elbow-Orthoses), Ellbogen-Schulter-Orthesen (Elbow-Shoulder-Orthoses), Rumpf-Schulter-Orthesen (Torso-Shoulder-Orthoses), Rumpf-Schulter-Ellbogen-Orthese (Torso-Shoulder-Elbow-Orthoses), Schulter-Orthesen (Shoulder-Orthoses), Prothese mit Oberschenkelhülsen (Prostheses with above knee socket) oder Prothesen mit Beckenkorb (Prostheses with pelvic socket).

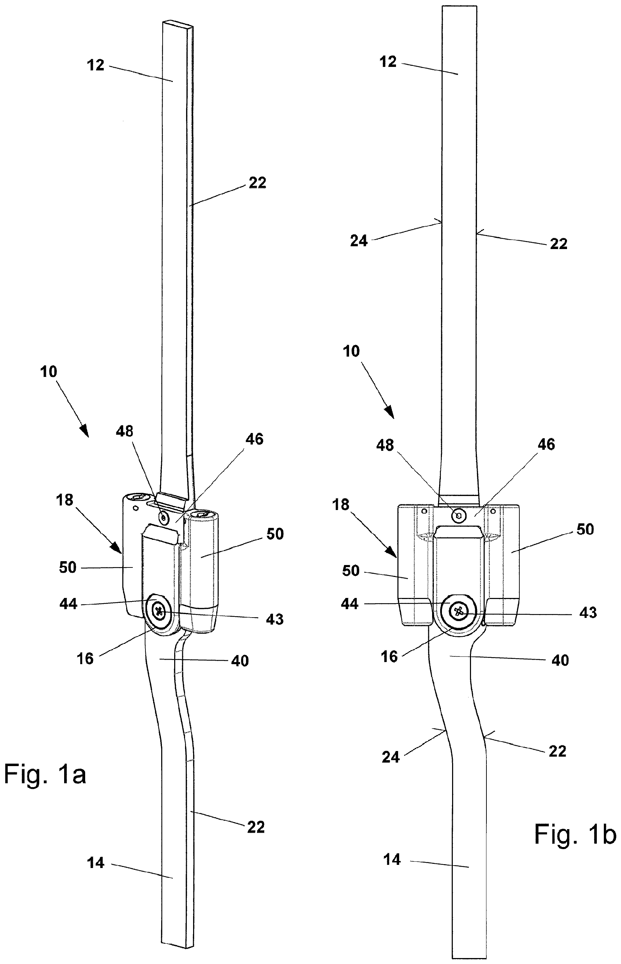

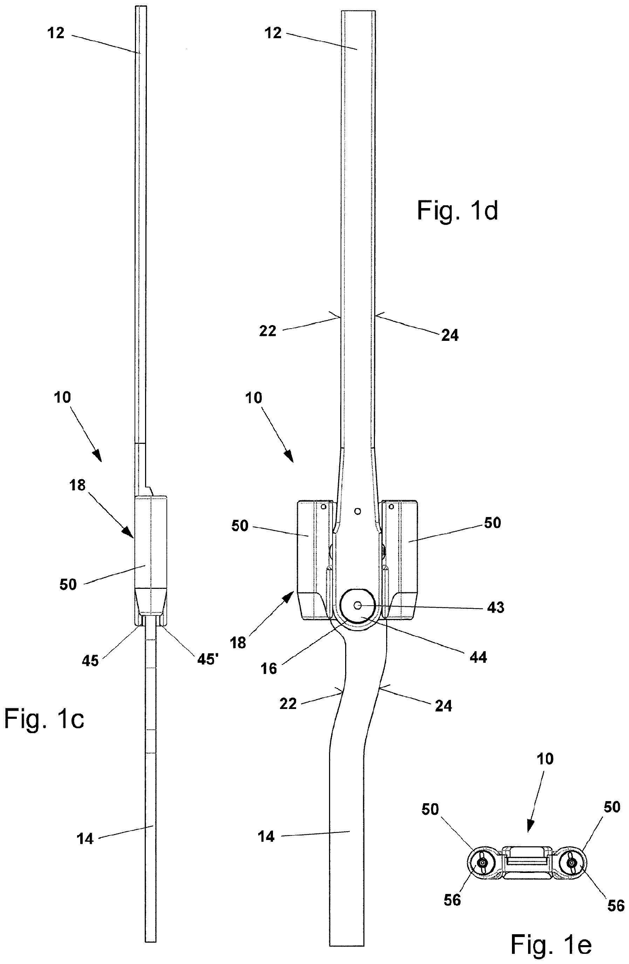

In den Fig. 1a bis 1e ist eine erste Ausführungsform eines Orthesengelenkes 10 nach der Erfindung dargestellt. Das Orthesengelenk 10 umfasst dabei ein Oberteil 12 bzw. eine proximale Komponente bzw. eine Orthesenschiene und einen Fußbügel 14 bzw. eine distale Komponente.

Das Oberteil 12 und der Fußbügel 14 sind miteinander über ein Drehgelenk 16 mit einer Drehachse zueinander relativ verschwenkbar verbunden.

Weiterhin umfasst das erfindungsgemäße Orthesengelenk 10 bei der Ausführungsform der Fig. 1a bis 1e ein Aufnahmeelement 18, in bzw. von welchem auf wenigstens einer Seite oder zu beiden Seiten des Drehgelenkes 16 ein federelastisches Funktionselement 20 aufgenommen ist. Durch das federelastische Funktionselement 20 ist der Fußbügel 14, wie noch näher dargestellt ist, dorsal und/oder plantar, d.h. im Bereich der dorsalen bzw. vorderen Seite bzw. Seitenkante 22 bzw. plantaren bzw. hinteren Seite bzw. Seitenkante 24, beaufschlagbar.

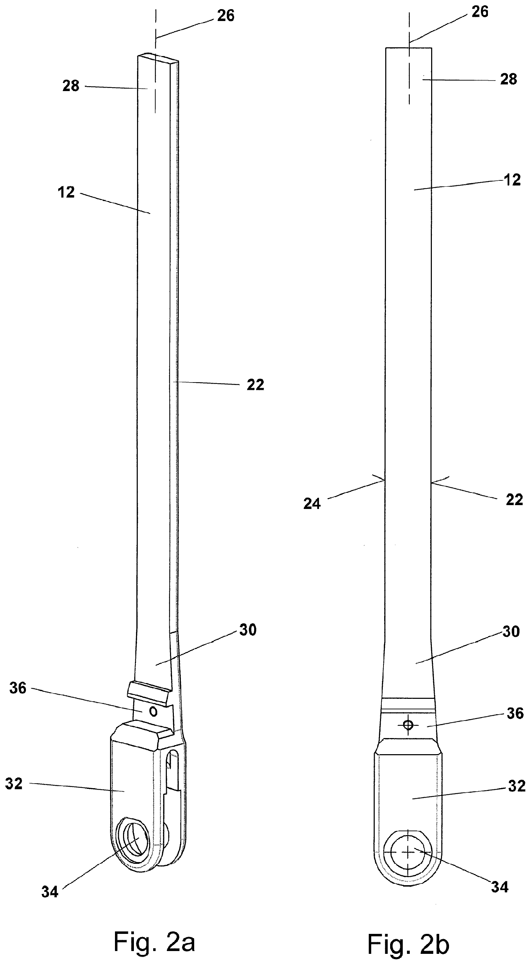

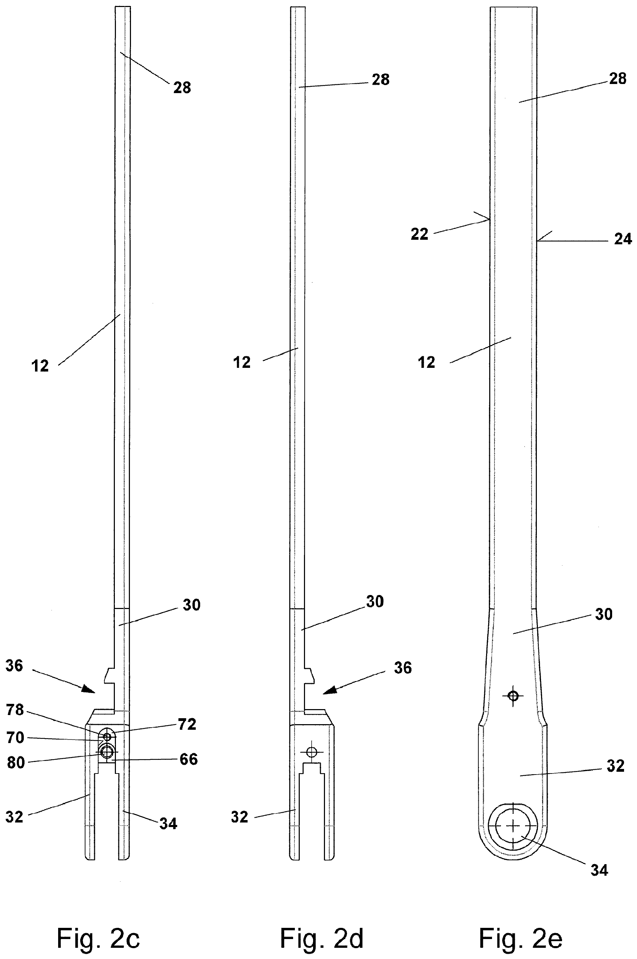

Wie in den Fig. 2a bis 2e in größerem Detail gezeigt ist, weist das Oberteil 12 eine Längserstreckung entsprechend der Längsachse 26 auf. Das Oberteil 12 ist einstückig bzw. einteilig bzw. integral ausgebildet.

Das obere Ende 28 des Oberteiles 12 ist in Form einer Schiene bzw. einer proximalen Komponente bzw. einer Orthesenschiene ausgebildet, die auf eine an den Unterschenkel des Patienten (nicht dargestellt) angepasste Länge abgelängt werden kann.

Das untere Ende 30 des Oberteiles 12 ist als Gabel 32 bzw. gabelförmig ausgebildet, um eine genaue, stabile und zuverlässige Aufnahme, Halterung und Drehung von Fußbügel 14 und Drehgelenk 16 bzw. deren Drehachse bzw. Achsschenkel (nicht gezeigt) in der Ausnehmung 34 sicherzustellen.

Das Oberteil 12 besteht mithin aus dem oberen Ende 28 und dem unteren Ende 30 und ist einstückig bzw. einteilig bzw. integral ausgebildet. Die eigentliche proximale Komponente bzw. Orthesenschiene und die Gabel 32, wobei Letztere einen Teil des Drehgelenkes 16 bildet, sind daher nicht - wie im Stand der Technik - als gesonderte, voneinander unabhängige und erst zusammenzufügende und miteinander zu verbindende bzw. zu befestigende Bauteile ausgebildet. Auf diese Weise lassen sich neben einer Vielzahl weiterer Vorteile gegenüber dem Stand der Technik die Anzahl von einzelnen Bauteilen erheblich vermindern, damit der Aufwand in Herstellung und Kosten wesentlich reduzieren und zugleich die Stabilität der erfindungsgemäßen Orthesengelenkes 10 sowie damit einhergehend das äußere Erscheinungsbild bzw. Design verbessern.

Weiterhin ist das Oberteil 12 zwischen dem oberen Ende 28 und dem unteren Ende 30, insbesondere oberhalb benachbart zu und/ oder geringfügig beabstandet von der Gabel 32, mit einer Nut 36 oder dergleichen Ausnehmung versehen. Die Nut 36 oder dergleichen Ausnehmung erstreckt sich im Wesentlichen quer, bevorzugt im Wesentlichen senkrecht oder senkrecht, zur Längsachse 26 des Oberteiles 12, und zwar in der Blattebene, wie in der Fig. 2b veranschaulicht, bzw. senkrecht zur Blattebene, wie in den Fig. 2c und 2d dargestellt ist. Insoweit verläuft die Nut 36 oder dergleichen Ausnehmung (in Richtung) von der dorsalen bzw. vorderen Seite bzw. Seitenkante 22 hin zu der plantaren bzw. hinteren Seite bzw. Seitenkante 24, und umgekehrt.

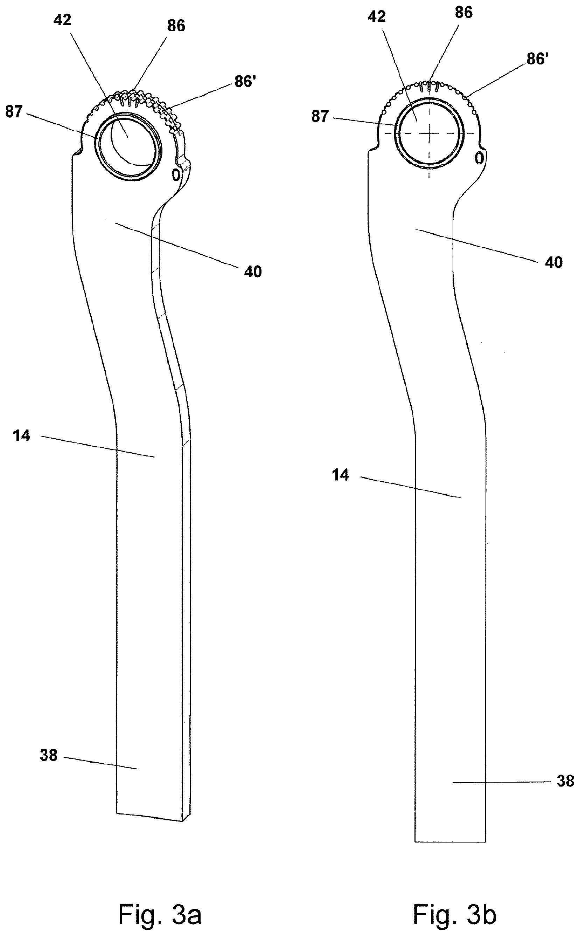

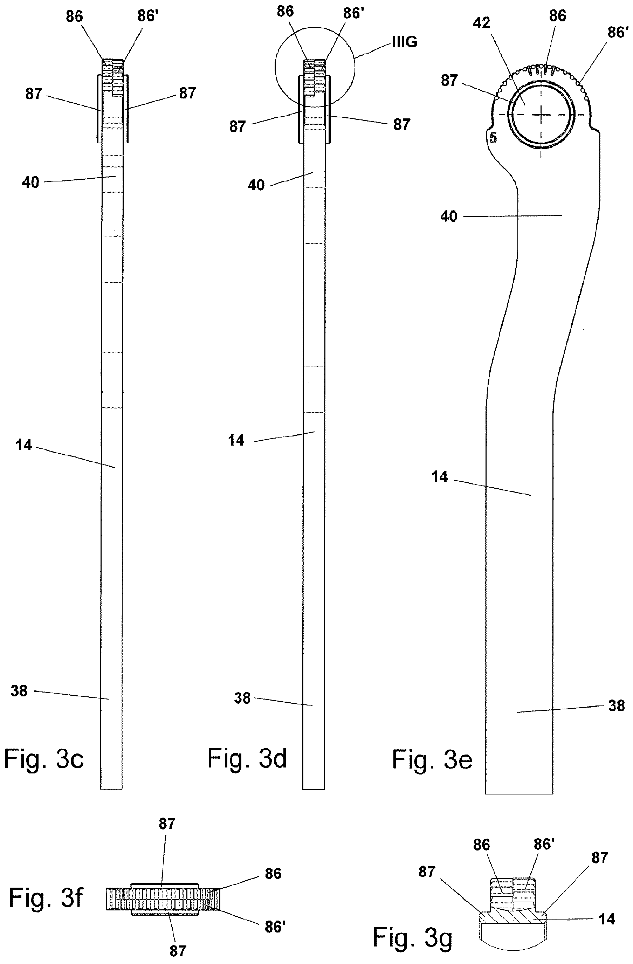

Wie in den Fig. 3a bis 3g in größerem Detail gezeigt ist, ist der Fußbügel 14 bzw. die distale Komponente ebenfalls länglich ausgebildet.

Das untere Ende 38 des Fußbügels 14 ist auch in Form einer Schiene bzw. Orthesenschiene ausgebildet, die auf eine an den Unterschenkel des Patienten (nicht dargestellt) angepasste Länge abgelängt werden kann.

Das obere Ende 40 des Fußbügels 14 ist mit einer Öffnung 42 bzw. einem Auge versehen, welche/s als Pendant zu der Ausnehmung 34 der Gabel 32 einen Teil des Drehgelenkes 16 bildet und zum Beispiel mit einem von der Ausnehmung 34 der Gabel 32 und von der Ausnehmung 42 des Fußbügels 14 aufgenommenen Achsschenkel oder dergleichen Lagerbolzen zusammenwirkt.

Entsprechend den Fig. 1a bis 1e sind Oberteil 12 und Fußbügel 14 miteinander über das Drehgelenk 16 bzw. den Achsschenkel oder dergleichen Lagerbolzen mittels einer Schraube 43 und zwei drehgesicherten Sicherungsscheiben 44 oder dergleichen nach deren Montage und Ausrichtung zueinander relativ verschwenkbar verbunden.

Der Fig. 1c ist darüber hinaus entnehmbar, dass zwischen Oberteil 12 und Fußbügel 14 im Bereich des Drehgelenkes 16 zwischen dem unteren Ende 30 des Oberteiles 12 und dem oberen Endes 40 des Fußbügels 16 plättchenförmige Zwischenschichten 45, 45' oder dergleichen Unterlegscheiben aus Kunststoff angeordnet sind. Als Kunststoff ist vorzugsweise ein selbstschmierender Kunststoff vorgesehen, um das erfindungsgemäße Orthesengelenk 10 leichtgängig zu machen und die Leichtgängigkeit im Betrieb aufrechtzuerhalten. Wie in der Fig. 1c zusätzlich angedeutet ist, können die plättchenförmige Zwischenschichten 45, 45' oder dergleichen Unterlegscheiben unterschiedliche Dicken aufweisen, um den Fußbügel 16 mittig zwischen der Gabel 32 des Oberteiles 12 und in Flucht zu dem einen oder den zwei Funktionselement/en 20 anzuordnen. Beispielsweise ist die Zwischenschicht 45 gegenüber der Zwischenschicht 45' um den Faktor 2 dicker ausgebildet.

Wie weiter insbesondere in den Fig. 1a bis 1e ersichtlich ist, ist das Aufnahmeelement 18 bei der Ausführungsform des erfindungsgemäßen Orthesengelenkes 10 ebenfalls einstückig bzw. einteilig bzw. integral ausgebildet.

Dabei umfasst das Aufnahmeelement 18 einen Befestigungsabschnitt 46. Der Befestigungsabschnitt 46 erstreckt sich von der dorsalen Seite bzw. Seitenkante 22 zur plantaren Seite bzw. Seitenkante 24, und umgekehrt, quer zur Längsachse 26 des Oberteiles 12. Wie beim Ausführungsbeispiel des erfindungsgemäßen Orthesengelenkes 10 nach den Fig. 1a bis 2e verwirklicht ist, erstreckt sich der Befestigungsabschnitt 46 in vorteilhafter Weise im Wesentlichen senkrecht oder senkrecht zur Längsachse 26 des Oberteiles 12. Ohne im Einzelnen dargestellt zu sein, kann sich der Befestigungsabschnitt 46 des Aufnahmeelementes 18 ebenso in einem Winkel schräg zur Längsachse 26 des Oberteiles 12 erstrecken.

Über den Befestigungsabschnitt 46 ist das Aufnahmeelement 18 an dem Oberteil 12 lösbar befestigbar.

Bevorzugt ist das Aufnahmeelement 18 an dem Oberteil 12 form- und/oder kraftschlüssig befestigbar. Das Aufnahmeelement 18, insbesondere der Befestigungsabschnitt 46 des Aufnahmeelementes 18, ist in die/der Nut 36 des Oberteiles 12 einsetzbar.

Durch die Aufnahme des Aufnahmeelementes 18 in die/der Nut 36 ist ein Formschluss erreicht, um auftretende Kräfte und/oder Momente unmittelbar auf das Oberteil 12 zu übertragen. Ein solcher Formschluss ist bei geringem oder ohne Spiel am wirksamsten.

Ohne im Einzelnen dargestellt zu sein, lässt sich durch eine seitliche Anlage oder Berührung des Aufnahmeelementes 18 an der dorsalen bzw. vorderen Seite bzw. Seitenkante 22 und/oder der plantaren bzw. hinteren Seite bzw. Seitenkante 24 des Oberteiles 12 ein zusätzlicher Formschluss erreichen, um auftretende Kräfte und/oder Momente unmittelbar auf Oberteil 12 zu übertragen. Ein solcher Formschluss ist bei geringem oder ohne Spiel am wirksamsten.

Wie darüber hinaus in den Fig. 1a bis 1e erkennbar ist, ist das Aufnahmeelement 18 bei der Ausführungsform des erfindungsgemäßen Orthesengelenkes 10 an dem Oberteil 12 wirksam über eine Schraubverbindung, nämlich eine Schraube 48, verbunden. Ohne im Einzelnen dargestellt zu sein, ist es ebenso denkbar, anstelle einer Schraubverbindung eine Rast-, Schnapp- oder dergleichen -verbindung vorzusehen.

Weiterhin umfasst das Aufnahmeelement 18 wenigstens einen Aufnahmeabschnitt 50, der mit dem Befestigungsabschnitt 46 einstückig bzw. einteilig bzw. integral ausgebildet ist.

Bei der in den Fig. 1a bis 1e gezeigten Ausführungsform des erfindungsgemäßen Orthesengelenkes 10 sind vorzugsweise zwei Aufnahmeabschnitte 50 vorgesehen. Die zwei Aufnahmeabschnitte 50 sind an dem Befestigungsabschnitt 46 bei dem gezeigten Ausführungsbeispiel im Wesentlichen parallel oder parallel zur Längsachse 26 des Oberteiles 12 angeordnet, vorliegend nämlich jeweils parallel zur Längsachse 26. Damit sind letztendlich auch die zwei Aufnahmeabschnitte 50 zueinander parallel angeordnet.

Ohne im Einzelnen dargestellt zu sein, ist es darüber hinaus jedoch möglich, den wenigstens einen Aufnahmeabschnitt 50 an dem Befestigungsabschnitt 46 in einem Winkel schräg verlaufend oder die zwei Aufnahmeabschnitte 50 an dem Befestigungsabschnitt 46 in anderer Weise symmetrisch, also zum Beispiel V-förmig zulaufend hin zu dem oberen Ende 28 oder hin zu dem unteren Ende 30 des Oberteiles 12 anzuordnen.

Schließlich ist erfindungsgemäß auch eine gemischte Anordnung der zwei Aufnahmeabschnitte 50 zueinander denkbar, also einmal parallel und einmal schräg verlaufend, d.h. in einem Winkel zur Längsachse 26 des Oberteiles 12 - sei es hin zu dem oberen Ende 28, sei es hin zu dem unteren Ende 30 des Oberteiles 12.

Der Befestigungsabschnitt 46 ist zwischen bzw. mittig zu den beiden Aufnahmeabschnitten 50 angeordnet. Bei diesem Ausführungsbeispiel handelt es sich mithin um einen mittleren Befestigungsabschnitt 50.

Die zwei Aufnahmeabschnitte 50 sind vorgesehen, um das/die Funktionselemente/s 20 seitlich des Oberteiles 12 aufzunehmen. Entsprechend den Fig. 1a bis 1e sind die zwei Aufnahmeabschnitte 50 daher seitlich und/oder parallel zu dem Orthesengelenk 10 und der dorsalen Seite bzw. Seitenkante 22 bzw. plantaren Seite bzw. Seitenkante 24 des Oberteiles 12 sowie diesen zugewandt angeordnet. Wie zuvor bereits ausgeführt, können die zwei Aufnahmeabschnitte 50 mit geringem Spiel oder sogar ohne Spiel zu der dorsalen Seite bzw. Seitenkante 22 und/oder plantaren Seite bzw. Seitenkante 24 des Oberteiles 12 angeordnet sein, um auftretende Kräfte und/oder Momente mittels Formschluss unmittelbar auf das Oberteil 12 zu übertragen. Bei Anlage bzw. Berührung der zwei Aufnahmeelemente 50 an der dorsalen Seite 22 und/oder plantaren Seite 24 des Oberteiles 12, d.h. bei fehlendem Spiel, ist ein solcher Formschluss am wirksamsten.

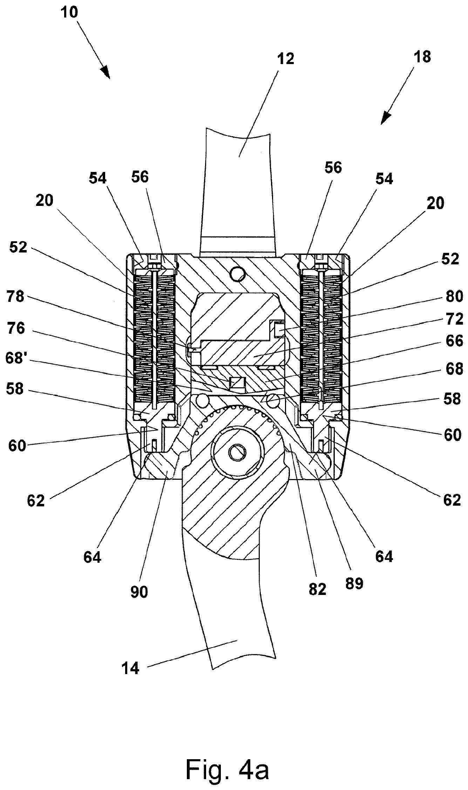

Wie sich den Fig. 1a bis 1e und vor allem der Fig. 4a entnehmen lässt, sind die zwei Aufnahmeabschnitte 50, die an dem mittleren Befestigungsabschnitt 46 des Aufnahmeelementes 18 angeformt sind, im Wesentlichen gekapselt ausgebildet. Eine solche verkapselte Ausgestaltung des Aufnahmeelementes 18 bringt eine Reihe von Vorteilen mit sich. So ist eine einfache, schnelle und individuelle Handhabung sowie Anpassung des Orthesengelenkes 10 bei der medizinischen und/oder therapeutischen Anwendung möglich, und zwar zu jeder Zeit der Therapie. Weiterhin lässt sich eine zusätzliche Standardisierung bzw. Normierung der einzelnen Bauteile erhalten. In dem gekapselten Aufnahmeelement können beliebig ausgestaltete Funktionselemente 20 eingebracht werden, ohne dessen äußere Form, Aussehen und Abmessung zu tangieren. Darüber wird einer Verschmutzung des/der Funktionselemente/s 20 entgegengewirkt und lässt sich eine unaufwendige Sauberhaltung des erfindungsgemäßen Orthesengelenkes insgesamt erreichen.

Entsprechend der Fig. 4a sind die zwei Aufnahmeabschnitte 50 jeweils mit einer länglichen Ausnehmung 52 oder Bohrung versehen, um ein zugeordnetes Funktionselement 20 aufnehmen zu können. Bei der in der Fig. 4a dargestellten Ausführungsform ist/sind das/die Funktionselement/e 20 als mindestens eine Feder, insbesondere Druckfeder, vorzugsweise Tellerfeder, bzw. einem daraus gebildeten Federpaket oder Tellerfederpaket ausgestaltet.

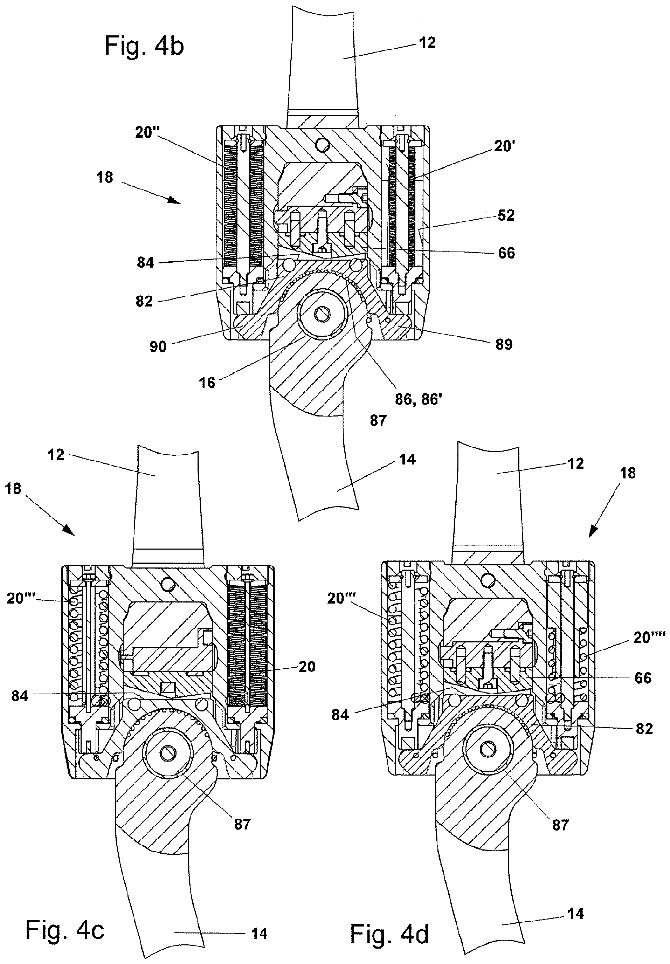

Die Ausführungsformen des erfindungsgemäßen Orthesengelenkes 10 der Fig. 4b bis 4d unterscheiden sich von derjenigen der Fig. 4a lediglich in der Wahl und Anordnung des/der Funktionselemente 20. Abgesehen davon, besteht jedoch Übereinstimmung.

So sind bei der Ausführungsform des erfindungsgemäßen Orthesengelenkes 10 nach der Fig. 4b anstatt von zwei Funktionselementen 20 in Form eines Tellerfederpaketes mit maximalen Durchmesser in der Ausnehmung 52 des dorsalen Aufnahmeabschnittes 50 ein Funktionselement 20' in Form eines Tellerfederpaketes mit minimalem Durchmesser angeordnet. In der Ausnehmung 52 des plantaren Aufnahmeabschnittes 50 ist ein Funktionselement 20" in Form eines Tellerfederpaketes mit mittlerem Durchmesser vorgesehen.

Bei der Ausführungsform des erfindungsgemäßen Orthesengelenkes 10 nach der Fig. 4c sind in der Ausnehmung 52 des dorsalen Aufnahmeabschnittes 50 ein Funktionselement 20 wiederum in Form eines Tellerfederpaketes mit maximalem Durchmesser und in der Ausnehmung 52 des plantaren Aufnahmeabschnittes 50 ein Funktionselement 20'" in Form einer Schraubenfeder von ganzer Länge angeordnet.

Bei der Ausführungsform des erfindungsgemäßen Orthesengelenkes 10 nach der Fig. 4d ist in der Ausnehmung 52 des dorsalen Aufnahmeabschnittes 50 ein Funktionselement 20"" in Form einer Schraubenfeder von halber Länge und in der Ausnehmung 52 des plantaren Aufnahmeabschnittes 50 ein Funktionselement 20'" in Form einer Schraubenfeder von ganzer Länge angeordnet.

Ohne im Einzelnen dargestellt zu sein, ist es möglich, das/die Funktionselement/e 20, 20', 20", 20'", 20"" kumulativ oder alternativ zu einer Feder, insbesondere Druckfeder, vorzugsweise Tellerfeder und/oder Schraubenfeder, auch als Kunststoffelement, insbesondere aus Polystyren, AcrylnitrilButadien-Styrol-Copolymeren, Acryl, Polycarbonat oder Gummi, oder eine Feder, insbesondere Zugfeder, vorzugsweise Schraubenfeder und/oder Ringfeder aus federelastischem Material, und/oder eine Gasdruckfeder und/oder eine Gaszugfeder, und/oder einen Dämpfer und/oder einen Motor, insbesondere Schrittmotor, und/oder eine magnetische Einrichtung, insbesondere ein Magnet, und/oder eine Kombination daraus und/oder einem daraus gebildeten Paket auszubilden.

Zurückkommend auf die Fig. 4a, umfassen die Aufnahmeabschnitte 50 des Weiteren jeweils eine Öffnung 54 zur Einbringung des zugeordneten Funktionselementes 20, 20', 20", 20'", 20"". Die Öffnung 54 ist vorzugsweise mittels Verschraubung, d.h. hier durch eine Schraube 56, verschließbar. Zugleich eignet sich die Schraube 56, Weg und Kraft des in die Ausnehmung 52 eingesetzten Funktionselementes 20, 20', 20", 20'", 20"" an die medizinischen und/oder therapeutischen Erfordernisse beliebig und/oder individuell anzupassen.

Darüber hinaus umfassen die zwei Aufnahmeabschnitte 50, wie der Fig. 4a ebenfalls entnehmbar ist, jeweils ein Betätigungselement 58, das einer dorsalen und/oder plantaren Beaufschlagung des Fußbügels 14 dient. Das Betätigungselement 58 ist in dem Aufnahmeabschnitt 50 axial verschiebbar und von dem Funktionselement 20, 20', 20", 20'", 20"" beaufschlagt.

Um den Fußbügel 14 dorsal und/oder plantar beaufschlagen zu können, erstreckt sich das Betätigungselement 58 teilweise durch eine Durchgangsbohrung 60 oder dergleichen Durchgang, die/der in Längsrichtung mit der länglichen Ausnehmung 52 kommuniziert. Das Betätigungselement 58 weist stirnseitig einen Stift 62 oder Bolzen auf, welcher die Durchgangsbohrung 60 durchgreift und mit dessen Stirnseite 64 mit dem Fußbügel 14 zu dessen dorsalen und/oder plantaren Beaufschlagung in Kontakt kommt bzw. steht.

Entsprechend den Fig. 2c und 4a ist dem Oberteil 12 ein Keilelement 66 zugeordnet, über welches die Bewegungsfreiheit des erfindungsgemäßen Orthesengelenkes 10 mittels wenigstens einer, vorzugsweise zwei, Keilfläche/n 68, 68' einstellbar ist. Das Oberteil 12 ist mit einer weiteren Nut 70 oder dergleichen Ausnehmung versehen, die sich im Wesentlichen senkrecht zur Längsachse 26 des Oberteiles 12 erstreckt, und zwar senkrecht zur Blattebene, wie in der Fig. 2c veranschaulicht, bzw. in der Blattebene, wie in der Fig. 4a dargestellt ist. Insoweit verläuft die Nut 70 oder dergleichen Ausnehmung in Richtung von der dorsalen bzw. vorderen Seite bzw. Seitenkante 22 hin zu der plantaren bzw. hinteren Seite bzw. Seitenkante 24, und umgekehrt. In die/der Nut 70 ist das Keilelement 66 einsetzbar und lösbar befestigt.

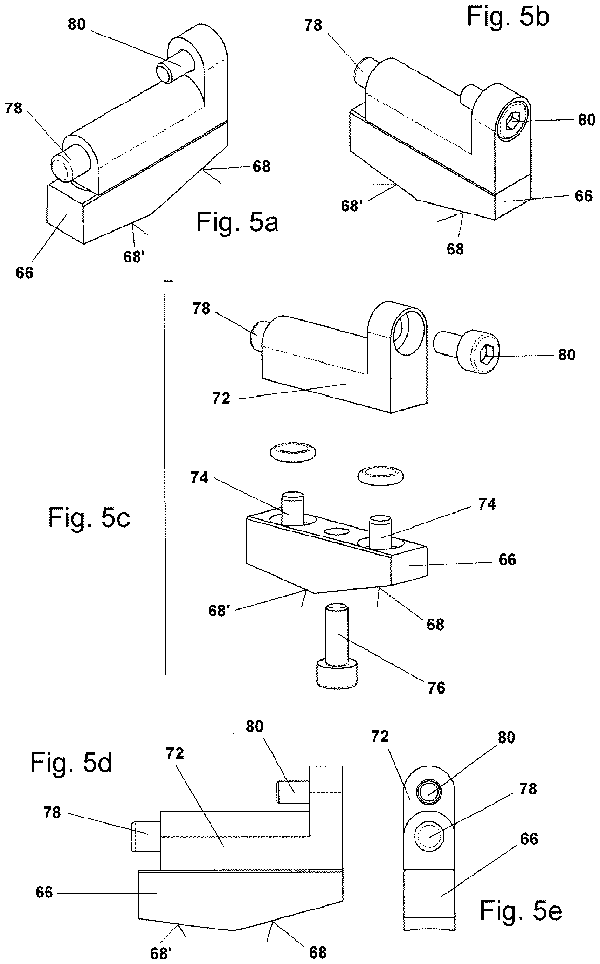

Das Keilelement 66 ist in den Fig. 5a bis 5e in größerem Detail gezeigt. Demnach weist das Keilelement 66 bei der gezeigten Ausführungsform zwei Keilflächen 68, 68' auf. Die Keilfläche 68 dient als dorsale Anschlagfläche. Die Keilfläche 68' ist als plantare Anschlagfläche vorgesehen.

Das Einsetzen des Keilelementes 66 in das Oberteil 12 kann unmittelbar (nicht gezeigt) erfolgen oder, wie aus den Fig. 5a bis 5e hervorgeht, mittelbar über ein Trägerelement 72 vorgenommen werden. Entsprechend den Fig. 5a bis 5e wird das Keilelement 66 an dem Trägerelement 72 mit zwei Zentrierstiften 74 und einer Schraube 76 befestigt. In der Nut 70 wird das Trägerelement 72 wiederum über einen Zentrierstift 78 und eine weitere Schraube 80 festgelegt.

Wie insbesondere die Fig. 4a bis 4d zeigen, ist dem Fußbügel 14 ein Stellelement 82 zugeordnet. Über das Stellelement 82 ist die Funktionsstellung des Orthesengelenkes 10 mittels einer Anschlagfläche 84 einstellbar. Die Anschlagfläche 84 ist der wenigstens einen, insbesondere den zwei, Keilfläche/n 68, 68' zugewandt und wirkt mit der/den Keilfläche/n 68, 68' zusammen.

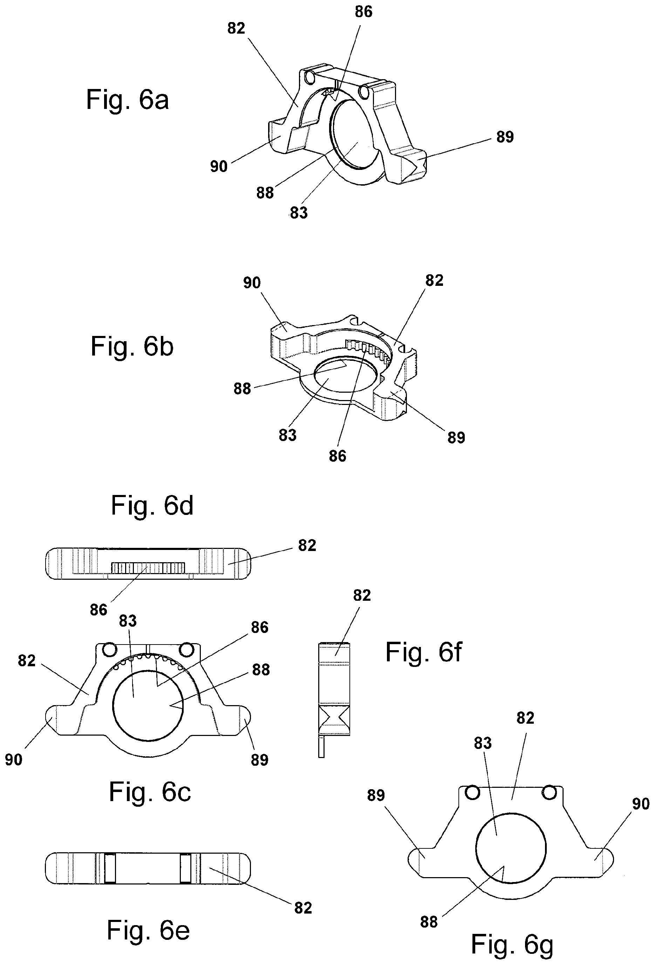

Der Fußbügel 14 ist mit dem Stellelement 82 lösbar verbunden. Zu diesem Zweck sind der Fußbügel 14 und das Stellelement 82, wie in den Fig. 3a bis 3g und 6a bis 6g dargestellt ist, mit einer Rast- oder Zahnverbindung 86 zur stufenverstellbaren Einstellung der Funktionsstellung des Orthesengelenkes 10 verbunden.

Die Rast- oder Zahnverbindung 86 zwischen Fußbügel 14 und Stellelement 82 ist in einer im Wesentlichen halbkreis- oder teilkreisförmigen Anordnung um das Drehgelenk 16 herum angeordnet. Bei dem Ausführungsbeispiel der Fig. 3a bis 3g ist die Rast- oder Zahnverbindung 86 über einen Bereich von nahezu 180° um das Drehgelenk 16 angeordnet. Bei dem in den Fig. 6a bis 6g dargestellten Ausführungsbeispiel umläuft die Rast- oder Zahnverbindung 86 einen Bereich von nahezu 90° um das Drehgelenk 16.

Die Rast- oder Zahnverbindung 86 weist zwischen Fußbügel 14 und Stellelement 82 eine gleichmäßige Teilung auf, durch welche die Funktionsstellung des Orthesengelenkes 10 in regelmäßigen Stufen einstellbar ist.

Entsprechend den Fig. 3a und 3c bis 3g ist der Fußbügel 14 vorzugsweise mit zwei Rast- oder Zahnverbindungen 86, 86' ausgestattet. Die zwei Rast- oder Zahnverbindungen 86, 86' erstrecken sich jeweils über etwa die Hälfte der Dicke bzw. Tiefe des Fußbügels 14. Deren Teilung stimmt dabei jeweils miteinander überein, ist jedoch relativ zueinander versetzt.

Das Stellelement 82 weist bei der Ausführungsform des erfindungsgemäßen Orthesengelenkes 10, wie aus den Fig. 6a bis 6g hervorgeht, eine Rast- oder Zahnverbindung 86 auf, deren Dicke bzw. Tiefe im Wesentlichen an die Dicke bzw. Tiefe einer der zwei Rast- oder Zahnverbindungen 86, 86' des Fußbügels 14 angepasst ist, also etwa der Hälfte der Dicke bzw. Tiefe des Fußbügels 14 entspricht. Hierdurch wird eine sehr feine Stufenverstellbarkeit des Stellelementes 82 und damit der Einstellung der Funktionsstellung des Orthesengelenkes 10 ermöglicht, ohne eine zu feine Rast- und Zahnverbindung mit damit einhergehend erhöhten Kosten herstellen zu müssen.

Durch den Versatz ist es möglich, beispielsweise bei Verbindung des Stellelementes 82 mit dem Fußbügel 14 von dessen Oberseite her (Fig. 3a, 3b) durch die eine Rast- und Zahnverbindung 86 eine Teilung von 0°, 10°, 20°, 30°, 40°, ..., und bei Verbindung des Stellelementes 82 mit dem Fußbügel 14 von dessen Unterseite her (Fig. 3e) durch die andere Rast- und Zahnverbindung 86' eine Teilung von 5°, 15°, 25°, 35°, 45°, ..., zur Verfügung zu haben. Zur besseren Handhabung in der Praxis sind die Oberseite mit "0", die Unterseite mit "5" sichtbar gekennzeichnet.

Die einzelnen Rast- oder Zahnelemente der Rast- oder Zahnverbindung 86, 86' von Fußbügel 14 und Stellelement 82 sind ineinander eingreifbar und kommen durch relative Verschiebung von Fußbügel 14 und Stellelement 82 - einmal von der Unterseite, einmal von der Oberseite des Fußbügels 14 her - flächenbündig zur gegenseitigen Anlage.

Um ein Auseinanderfallen von Fußbügel 14 und Stellelement 82 nach gegenseitiger Ineingriffbringung und somit gegenseitiger flächenbündiger bzw. flächiger Anlage der Rast- und Zahnverbindung 86, 86' zu vermeiden, ist der Fußbügel 14 beidseitig mit einem ringförmigen, um die Ausnehmung 42 wenigstens teilweise umlaufenden Vorsprung 87 oder dergleichen Wulst versehen. Bei dem vorliegenden Ausführungsbeispiel ist der ringförmige Vorsprung 87 vollständig um die Ausnehmung 42 umlaufend ausgebildet und fasst damit die Ausnehmung 42 vollständig ein.

Der ringförmige Vorsprung 87 kommt mit der Innenwandung 88 der kreisförmigen Ausnehmung 83 des Stellelementes 82 entsprechend Fig. 6a bis 6g zur Anlage. Die runde Ausnehmung 83 des Stellelementes 82 weist einen Durchmesser auf, welcher dem Durchmesser der Ausnehmung 34 bzw. dem Außendurchmesser des Achsschenkels oder dergleichen Lagerbolzens des Drehgelenkes 16 zu dessen, insbesondere spielfreien, Aufnahme zuzüglich der radialen Breite bzw. Dicke des ringförmigen Vorsprunges 87 des Fußbügels 14 entspricht.

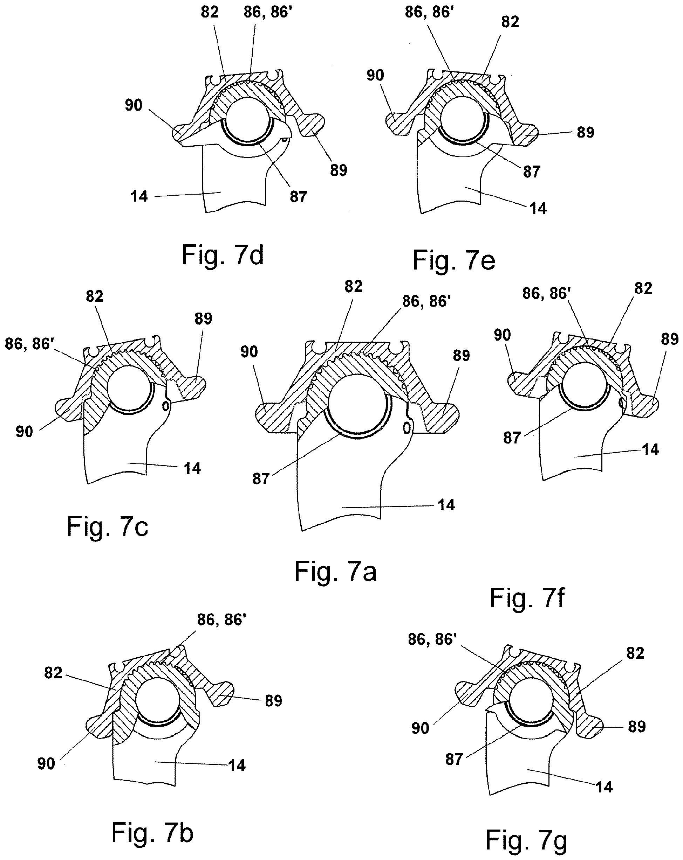

Wie in den Fig. 7a bis 7g schematisch dargestellt ist, lassen sich auf diese Weise beliebige Stufeneinstellungen des Stellelementes 82 gegenüber dem Fußbügel 14 vornehmen, mit geringem Herstellungsaufwand und ausreichender Dimensionierung.

Bei der Ausführungsform des erfindungsgemäßen Orthesengelenkes 10 entsprechend den Fig. 1a bis 1e umfasst der Fußbügel 14 zu beiden Seiten des Drehgelenkes 16 weiter einen dorsalen Vorsprung 89 und einen plantaren Vorsprung 90 oder dergleichen Auskragung, der/die durch das federelastische Funktionselement 20, 20', 20'', 20'', 20''', 20"" beaufschlagbar ist, welches in/von dem Aufnahmeelement 18 aufgenommen ist.

Der dorsale und der plantare Vorsprung 89, 90 des Fußbügels 14 zu beiden Seiten des Drehgelenkes 16 ist bei der Ausführungsform des erfindungsgemäßen Orthesengelenkes 10 in bevorzugter Weise einstückig an dem Stellelement 82 selbst angeformt.

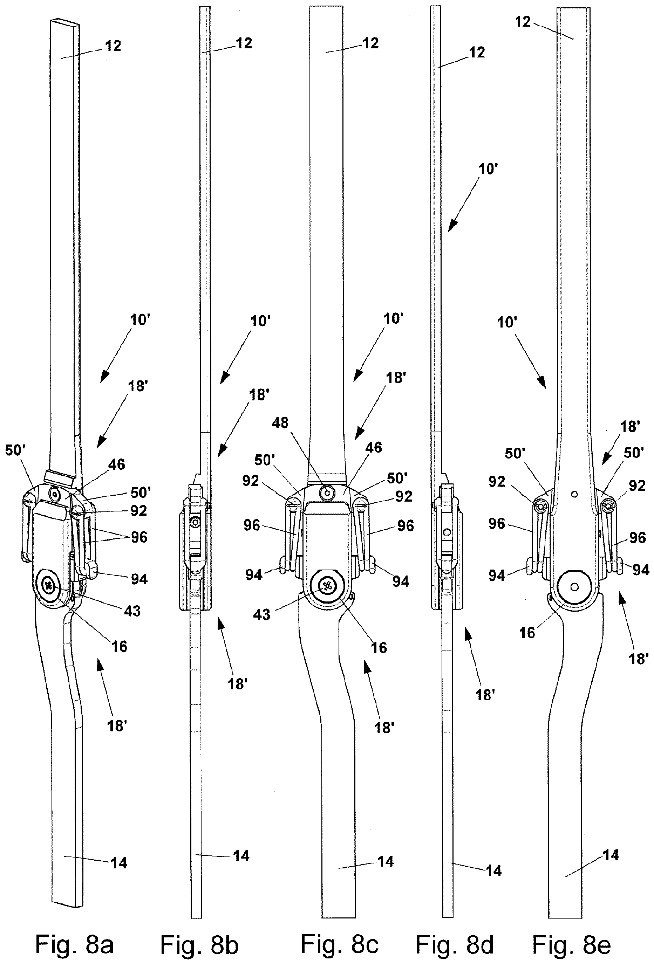

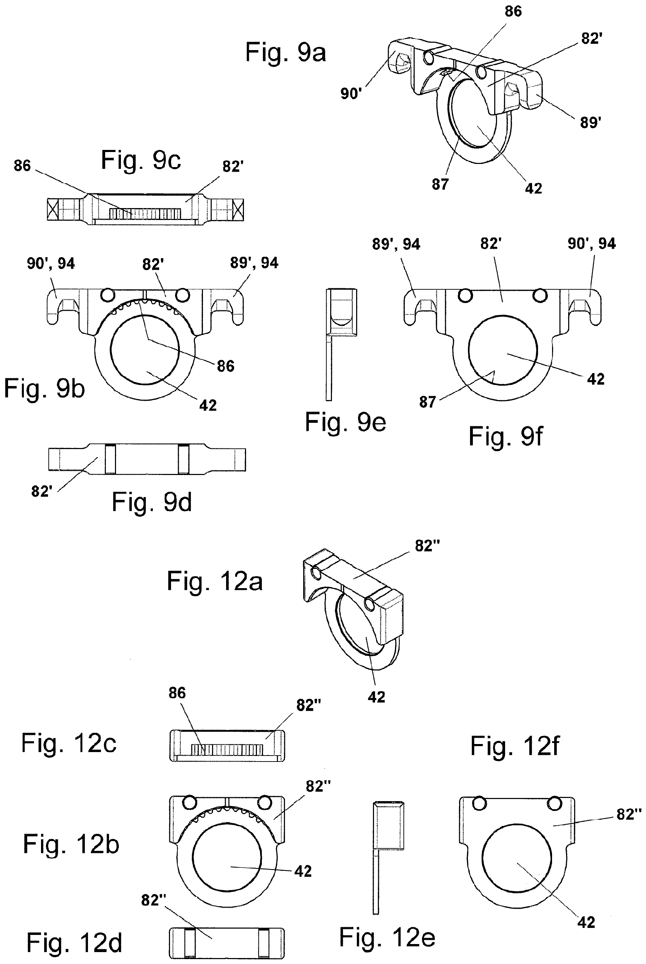

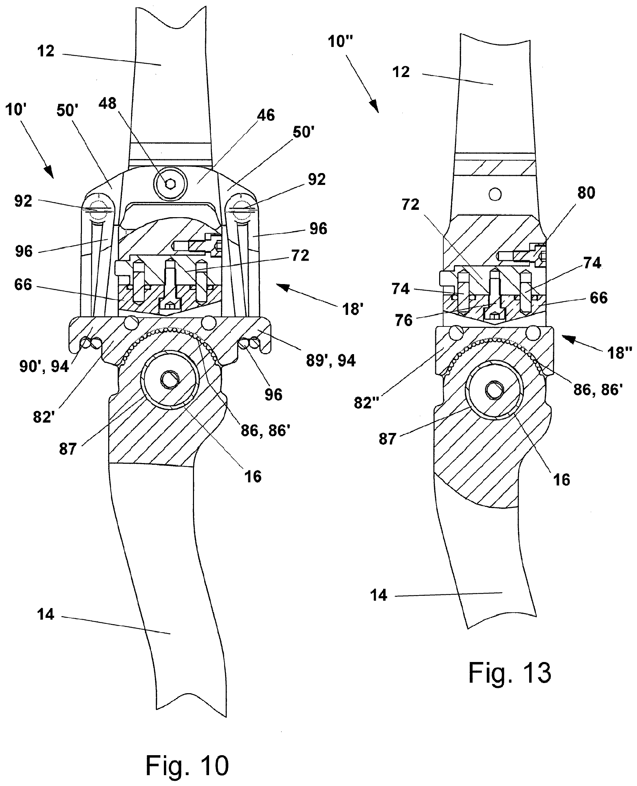

In den Fig. 8a bis 10 ist eine weitere Ausführungsform eines erfindungsgemäß ausgebildeten Orthesengelenkes 10' vorgestellt. Diese Ausführungsform unterscheidet sich von derjenigen der Fig. 1a bis 7g lediglich in der konstruktiven Ausgestaltung von Aufnahmeelement 18' und Stellelement 82'.

Demnach ist das Aufnahmeelement 18' durch zwei an dem Befestigungsabschnitt 46 angeformte Aufnahmeabschnitte 50', die nicht gekapselt, sondern im Gegenteil im Wesentlichen freiliegend ausgebildet sind, gebildet. Die zwei Aufnahmeabschnitte 50' sind jeweils mit Schrauben 92 oder sonstigen Vorsprüngen zur Aufnahme und Halterung des Funktionselementes 20, 20', 20", 20", 20'", 20"" versehen.

Der dorsale Vorsprung 89' und der plantare Vorsprung 90' des Stellelementes 82' sind jeweils in Form eines Hakens 94, einer Schraube, einer nutförmigen Ausnehmung oder dergleichen hakenförmig ausgebildeten kragenden Endes ausgestaltet.

Auf diese Weise lässt/lassen sich als Funktionselement/e 20 mindestens eine Feder, insbesondere eine Zugfeder 96, vorzugsweise eine Ringfeder aus federelastischem Material, wie Gummi, einsetzen. Dabei wird die Zugfeder 96 jeweils endseitig um bzw. über die Schrauben 92 und im mittleren Bereich über die Haken 94 gehalten. Die Zugfedern 96 verlaufen somit jeweils freiliegend, seitlich und parallel zu der dorsalen Seite 22 und zur plantaren Seite 24 des Orthesengelenkes 10.

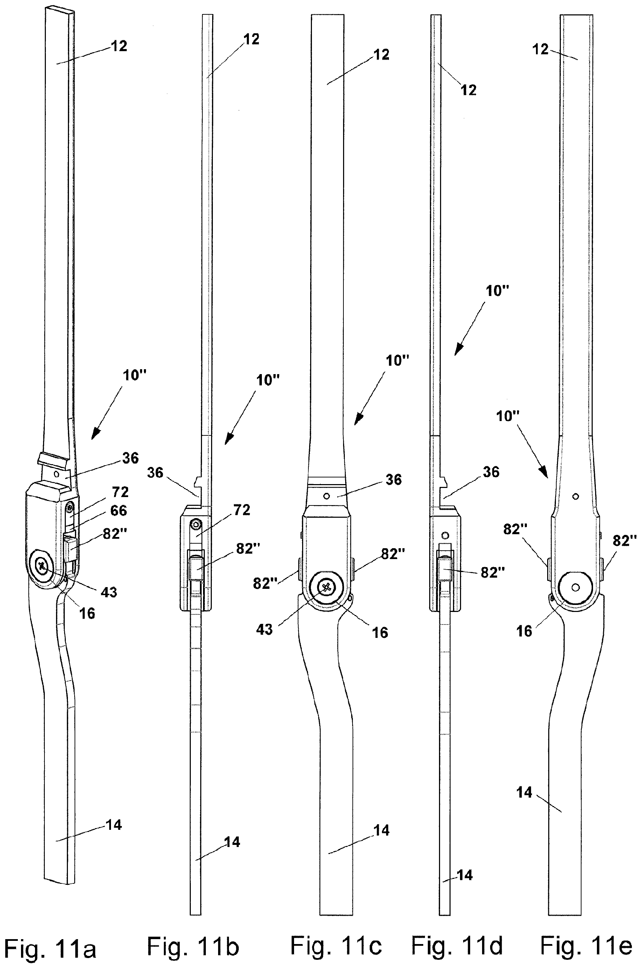

In den Fig. 11a bis 13 ist schließlich eine noch andere Ausführungsform eines erfindungsgemäß ausgebildeten Orthesengelenkes 10" vorgestellt. Diese Ausführungsform unterscheidet sich von denjenigen der Fig. 1a bis 7g und Fig. 8a bis 10 nur mehr in der konstruktiven Ausgestaltung des Stellelementes 82".

Demnach ist kein Aufnahmeelement 18, 18' vorhanden bzw. in der Nut 36 eingesetzt und an dem Oberteil 12 befestigt. Das Stellelement 82" ist ohne dorsalen Vorsprung und ohne plantaren Vorsprung ausgebildet.

Das erfindungsgemäße Orthesengelenk 10" kommt mithin ohne Funktionselement/e 20, 20', 20", 20", 20'", 20"" zur Anwendung, wobei dieses Orthesengelenk 10' quasi die Grundausstattung für ein Baukastensystem zur Bildung eines Orthesengelenkes 10, 10', 10" darstellt und bildet.

Das erfindungsgemäße Orthesengelenk 10, 10', 10" eignet sich mithin in besonders vorteilhafter Weise für ein Baukastensystem. Dabei ist eine beliebige, individuelle und eng sowie zeitnah bzw. sogar in Ist-Zeit auf ein sich (ständig) veränderndes Krankheitsbild abgestimmte Auswahl zwischen den unterschiedlich ausgebildeten Aufnahmeelementen 18, 18', d.h. dem Aufnahmeelement 18 in quasi verkapselter Form entsprechend der Ausführungsform des erfindungsgemäßen Orthesengelenkes 10 nach den Fig. 1a bis 7g oder wahlweise dem Aufnahmeelement 18' in quasi freiliegender Form entsprechend der Ausführungsform des erfindungsgemäßen Orthesengelenkes 10' nach den Fig. 8a bis 10 ermöglicht. Selbst wenn zwischenzeitlich oder nachträglich eine andere medizinische oder therapeutische Anwendung erforderlich werden würde, ließe sich wahlweise ebenso auf das Aufnahmeelement 18" in quasi der Grund- oder Basisform entsprechend der Ausführungsform des erfindungsgemäßen Orthesengelenkes 10" nach den Fig. 11a bis 13 zurückgreifen.

Damit lässt sich ein modularer Aufbau des erfindungsgemäßen Orthesengelenkes 10, 10', 10" einfach verwirklichen und eine enorme, bis dato im Stand der Technik nicht gekannte Vielseitigkeit des erfindungsgemäßen Orthesengelenkes 10, 10', 10" erhalten. Folglich ist neben einer einfachen, schnellen und individuellen Handhabung sowie - auch jederzeit, d.h. etwa nachträglichen - Anpassung des Orthesengelenkes 10, 10', 10" nach der Erfindung bei der medizinischen und/oder therapeutischen Anwendung eine wesentliche Standardisierung bzw. Normierung der einzelnen Bauteile ermöglicht.

Die Erfindung ist nicht auf die dargestellten Ausführungsformen des erfindungsgemäßen Orthesengelenkes 10, 10', 10" beschränkt. So ist es ohne weiteres möglich, die verschiedenen Ausführungsformen des erfindungsgemäßen Orthesengelenkes 10, 10', 10" untereinander bzw. miteinander beliebig zu kombinieren. Des Weiteren ist es denkbar, dass das Aufnahmeelement 18, 18' lediglich den Befestigungsabschnitt 46 und ein an dem Befestigungsabschnitt 46 angeordneten bzw. einstückig angeformten Aufnahmeabschnitt 50, 50' umfasst. Eine solche bauliche Ausgestaltung kann insbesondere von Vorteil sein, wenn dies aus medizinischen und/oder therapeutischen Gründen für nicht erforderlich erachtet wird und zugleich ästhetische Gesichtspunkte in den Vordergrund gerückt sind. Deutlich bevorzugt - nicht zuletzt aufgrund der sich daraus ergebenden Vielseitigkeit und Variationsvielfalt - ist allerdings ein Aufnahmeelement 50, 50' mit zwei an dem Befestigungsabschnitt 46 angeordneten Aufnahmeabschnitten 50, 50', die sich insbesondere jeweils parallel und/oder symmetrisch zur Längsachse 26 des Oberteiles 12, mithin beidseitig dazu, erstrecken. In diesem Fall ist der Befestigungsabschnitt 46 etwa mittig zu den zwei Aufnahmeabschnitten 50, 50' angeordnet. Der Befestigungsabschnitt 46 ist ein mittlerer Befestigungsabschnitt 46.

Ohne im Einzelnen dargestellt zu sein, ist es weiter auch denkbar, die Ausführungsformen der Aufnahmeelemente 18, 18' nach den Fig. 1a bis 7g bzw. 8a bis 9f zu kombinieren, also auf der dorsalen Seite 22 einen Aufnahmeabschnitt 50 bzw. auf der plantaren Seite 24 einen Aufnahmeabschnitt 50' vorzusehen, derart, dass ein Funktionselement 20 auf einer Seite gekapselt und auf der anderen Seite freiliegend angeordnet ist, und umgekehrt.

Darüber hinaus kann das Drehgelenk 16, welches das Oberteil 12 und den Fußbügel 16 miteinander drehbar verbindet, auch von jeder anderen üblichen konstruktiven Ausgestaltung sein. So hat sich zwar insbesondere eine Gabel 32 bzw. eine gabelförmige Ausbildung des Drehgelenkes 32 in der Praxis zur gleichmäßigen Kraft- und Momentenaufnahme bzw. -einleitung als besonders vorteilhaft für das erfindungsgemäße Orthesengelenk 10 erwiesen, ohne letztlich auf eine solche konstruktive Ausgestaltung beschränkt zu sein.

Schließlich ist das erfindungsgemäße Orthesengelenk 10 nicht darauf beschränkt, dass die Nut 36 oder dergleichen Ausnehmung 36 in/an dem Oberteil 12 zwischen dem oberen Ende 28 und dem unteren Ende 30, insbesondere oberhalb benachbart zu und/oder geringfügig beabstandet von der Gabel 32, senkrecht oder im Wesentlichen senkrecht zur Längsachse 26 des Oberteiles 12 angeordnet ist. Anstelle dessen kann die Nut 36 oder dergleichen Ausnehmung 36 in äquivalenter Weise, bei etwaiger Anpassung von weiteren Bauteilen, wie des Aufnahmeelementes 18, in/an dem Oberteil 12 zur Längsachse 26 des Oberteiles 12 auch in einem Winkel schräg verlaufend angeordnet werden.

## Patentansprüche

1. Orthesengelenk mit einem Oberteil (12) und einem Fußbügel (14), die miteinander über ein Drehgelenk (16) mit einer Drehachse zueinander relativ verschwenkbar verbunden sind, wobei der Fußbügel (14) zu beiden Seiten des Drehgelenkes (16) durch ein in/von einem Aufnahmeelement (18, 18') aufgenommenes, federelastisches Funktionselement (20, 20', 20", 20'", 20"") dorsal und/oder plantar beaufschlagbar ist, **dadurch gekennzeichnet, dass** das Aufnahmeelement (18, 18') über einen sich von einer dorsalen Seite (22) zu einer plantaren Seite (24), und umgekehrt, quer zu einer Längsachse (26) des Oberteiles (12) erstreckenden Befestigungsabschnitt (46) des Aufnahmeelementes (18, 18') an dem Oberteil (12) lösbar befestigbar ist und wenigstens einen, insbesondere zwei, an dem Befestigungsabschnitt (46) angeordnete/n Aufnahmeabschnitt/e (50, 50') zur Aufnahme des/der Funktionselemente/s (20, 20', 20", 20'", 20"") seitlich des Oberteiles (12) umfasst.

2. Orthesengelenk nach Anspruch 1, **dadurch gekennzeichnet, dass** sich der Befestigungsabschnitt (46) des Aufnahmeelementes (18, 18') von der dorsalen Seite (22) zur plantaren Seite (24), und umgekehrt, im Wesentlichen senkrecht oder senkrecht oder in einem Winkel schräg zur Längsachse (26) des Oberteiles (12) erstreckt.

3. Orthesengelenk nach Anspruch 1 oder 2, **dadurch gekennzeichnet, dass** das Aufnahmeelement (18, 18') mit dem Befestigungsabschnitt (46) und dem wenigstens einen, insbesondere den zwei, an dem Befestigungsabschnitt (46) angeordneten Aufnahmeabschnitt/en (50, 50') einstückig ausgebildet ist.

4. Orthesengelenk nach einem der Ansprüche 1 bis 3, **dadurch gekennzeichnet, dass** das Oberteil (12), an dem das Aufnahmeelement (18, 18') lösbar befestigt ist, einstückig ausgebildet ist.

5. Orthesengelenk nach einem der Ansprüche 1 bis 4, **dadurch gekennzeichnet, dass** das Aufnahmeelement (18, 18') an dem Oberteil (12) form- und/oder kraftschlüssig befestigbar ist.

6. Orthesengelenk nach einem der Ansprüche 1 bis 5, **dadurch gekennzeichnet, dass** das Oberteil (12) mit einer sich quer, insbesondere im Wesentlichen senkrecht oder senkrecht oder in einem Winkel schräg, zur Längsachse (26) des Oberteiles (12) erstreckenden Nut (36) oder dergleichen Ausnehmung versehen ist, in welche das Aufnahmeelement (18, 18'), insbesondere der Befestigungsabschnitt (46) des Aufnahmeelementes (18, 18'), vorzugsweise spielfrei, einsetzbar ist.

7. Orthesengelenk nach einem der Ansprüche 1 bis 6, **dadurch gekennzeichnet, dass** das Aufnahmeelement (18, 18') an dem Oberteil (12) über eine Schraub-, Rast-, Schnapp- oder dergleichen -verbindung (48) befestigbar ist.

8. Orthesengelenk nach einem der Ansprüche 1 bis 7, **dadurch gekennzeichnet, dass** der wenigstens eine, insbesondere die zwei, an dem Befestigungsabschnitt (46) angeformte/n Aufnahmeabschnitt/e (50) des Aufnahmeelementes (18, 18') parallel und/oder in einem Winkel schräg zur Längsachse (26) des Oberteiles (12) und/oder symmetrisch beidseits der Längsachse (26) des Oberteiles (12) und zueinander angeordnet ist/sind.

9. Orthesengelenk nach einem der Ansprüche 1 bis 8, **dadurch gekennzeichnet, dass** der wenigstens eine, insbesondere die zwei, an dem Befestigungsabschnitt (46) angeformte/n Aufnahmeabschnitt/e (50) des Aufnahmeelementes (18, 18') an der/die dorsale/n bzw. vordere/n Seite bzw. Seitenkante (22) und/oder der/die plantare/n bzw. hintere/n Seite bzw. Seitenkante (24) des Oberteiles (12) seitlich anliegen oder berühren.

10. Orthesengelenk nach einem der Ansprüche 1 bis 9, **dadurch gekennzeichnet, dass** der wenigstens eine, insbesondere die zwei, an dem Befestigungsabschnitt (46) angeformte/n Aufnahmeabschnitt/e (50) des Aufnahmeelementes (18, 18') im Wesentlichen gekapselt ausgebildet ist/sind.

11. Orthesengelenk nach einem der Ansprüche 1 bis 10, **dadurch gekennzeichnet, dass** der wenigstens eine, insbesondere die zwei, an dem Befestigungsabschnitt (46) angeformte/n Aufnahmeabschnitt/e (50) jeweils mit einer länglichen Ausnehmung (52) oder Bohrung zur Aufnahme des Funktionselementes (20, 20', 20", 20'", 20"") versehen ist/sind.

12. Orthesengelenk nach einem der Ansprüche 1 bis 11, **dadurch gekennzeichnet, dass** der wenigstens eine, insbesondere die zwei, an dem Befestigungsabschnitt (46) angeformte/n Aufnahmeabschnitt/e (50, 50') eine, insbesondere mittels Verschraubung (56), verschließbare Öffnung (54) zur Einbringung des Funktionselementes (20, 20', 20", 20'", 20"") umfasst/umfassen.

13. Orthesengelenk nach einem der Ansprüche 1 bis 12, **dadurch gekennzeichnet, dass** der wenigstens eine, insbesondere die zwei, an dem Befestigungsabschnitt (46) angeformte/n Aufnahmeabschnitt/e (50) ein in dem Aufnahmeabschnitt (50) axial verschiebbares und von dem Funktionselement (20, 20', 20", 20'", 20"") beaufschlagtes Betätigungselement (58) zur dorsalen und/oder plantaren Beaufschlagung des Fußbügels (14) umfasst/umfassen.

14. Orthesengelenk nach einem der Ansprüche 1 bis 13, **dadurch gekennzeichnet, dass** der wenigstens eine, insbesondere die zwei, an dem Befestigungsabschnitt (46) angeformte/n Aufnahmeabschnitt/e (50) ein von dem Funktionselement (20, 20', 20", 20'", 20"") beaufschlagtes und sich teilweise durch eine Durchgangsbohrung (60) oder dergleichen Durchgang erstreckendes Betätigungselement (58) zur dorsalen und/oder plantaren Beaufschlagung des Fußbügels (14) umfasst/umfassen.

15. Orthesengelenk nach einem der Ansprüche 1 bis 14, **dadurch gekennzeichnet, dass** das Betätigungselement (58) zur dorsalen und/oder plantaren Beaufschlagung des Fußbügels (14) durch ein dem Funktionselement (20, 20', 20", 20'", 20"") beaufschlagt und mit einem sich durch die Durchgangsbohrung (60) oder dergleichen Durchgang erstreckenden Stift (62) oder Bolzen gebildet ist.

16. Orthesengelenk nach einem der Ansprüche 1 bis 15, **dadurch gekennzeichnet, dass** der wenigstens eine, insbesondere die zwei, an dem Befestigungsabschnitt (46) angeformte/n Aufnahmeabschnitt/e (50') im Wesentlichen freiliegend ausgebildet ist/sind.

17. Orthesengelenk nach einem der Ansprüche 1 bis 16, **dadurch gekennzeichnet, dass** der wenigstens eine, insbesondere die zwei, an dem Befestigungsabschnitt (46) angeformte/n Aufnahmeabschnitt/e (50') mit Schrauben (92), Vorsprüngen oder nutförmigen Ausnehmungen zur Aufnahme und Halterung des Funktionselementes (20, 20', 20", 20'", 20"") versehen ist/sind.

18. Orthesengelenk nach einem der Ansprüche 1 bis 17, **dadurch gekennzeichnet, dass** dem Oberteil (12) ein Keilelement (66), über welches die Bewegungsfreiheit des Orthesengelenkes (10, 10', 10") mittels wenigstens einer, vorzugsweise zwei, Keilfläche/n (68, 68') einstellbar ist, zugeordnet ist.

19. Orthesengelenk nach Anspruch 18, **dadurch gekennzeichnet, dass** das Oberteil (12) mit einer sich im Wesentlichen senkrecht zur Längsachse (26) des Oberteiles (12) erstreckenden Nut (70) oder dergleichen Ausnehmung versehen ist, in welche das Keilelement (66), unmittelbar oder mittelbar über ein Trägerelement (72), einsetzbar und lösbar befestigt ist.

20. Orthesengelenk nach einem der Ansprüche 1 bis 19, **dadurch gekennzeichnet, dass** der Fußbügel (14) zu beiden Seiten des Drehgelenkes (16) einen dorsalen und einen plantaren Vorsprung (89, 89'; 90, 90') oder dergleichen Auskragung umfasst, der/die durch das in/von einem Aufnahmeelement (18, 18') aufgenommene, federelastische Funktionselement (20, 20', 20", 20'", 20"") beaufschlagbar ist.

21. Orthesengelenk nach einem der Ansprüche 18 bis 20, **dadurch gekennzeichnet, dass** dem Fußbügel (14) ein Stellelement (82, 82', 82"), über welches die Funktionsstellung des Orthesengelenkes (10, 10', 10") mittels einer der wenigstens einen Keilfläche (68, 68') zugewandten und mit der wenigstens einen Keilfläche (68, 68') zusammenwirkenden Anschlagfläche (84) einstellbar ist, zugeordnet ist, wobei der dorsale und der plantare Vorsprung (89, 89'; 90, 90') oder dergleichen Auskragung des Fußbügels (14) zu beiden Seiten des Drehgelenkes (16) einstückig an dem Stellelement (82, 82', 82") angeformt ist.

22. Orthesengelenk nach einem der Ansprüche 1 bis 21, **dadurch gekennzeichnet, dass** das/die Funktionselement/e (20, 20', 20", 20'", 20"") als mindestens eine Feder, insbesondere Druckfeder, vorzugsweise Tellerfeder (20, 20', 20") und/oder Schraubenfeder (20'", 20"") und/oder Kunststoffelement, oder insbesondere Zugfeder, vorzugsweise Schraubenfeder und/oder Ringfeder (96) aus federelastischem Material, und/oder eine Gasdruckfeder und/oder eine Gaszugfeder, und/oder einen Dämpfer und/oder einen Motor, insbesondere Schrittmotor, und/oder einer magnetischen Einrichtung, insbesondere einen Magneten, und/oder einer Kombination daraus und/oder einem daraus gebildeten Paket, ausgebildet ist/sind.

23. Baukastensystem zur Bildung eines Orthesengelenkes nach einem der Ansprüche 1 bis 22, umfassend ein Oberteil (12), einen Fußbügel (14), die miteinander über ein Drehgelenk (16) mit einer Drehachse zueinander relativ verschwenkbar verbunden sind, ein Aufnahmeelement (18, 18') und ein in/von dem Aufnahmeelement (18, 18') aufnehmbares, federelastisches Funktionselement (20, 20', 20", 20'", 20""), wobei das Aufnahmeelement (18, 18') über einen sich von einer dorsalen Seite (22) zu einer plantaren Seite (24), und umgekehrt, quer zu einer Längsachse (26) des Oberteiles (12) erstreckenden Befestigungsabschnitt (46) des Aufnahmeelementes (18, 18') an dem Oberteil (12) lösbar befestigbar ist und wenigstens einen, insbesondere zwei, an dem Befestigungsabschnitt (46) angeordnete/n Aufnahmeabschnitt/e (50, 50') zur Aufnahme des/der Funktionselemente/s (20, 20', 20", 20'", 20"") seitlich des Oberteiles (12) umfasst.

24. Verwendung eines Orthesengelenkes nach einem der Ansprüche 1 bis 23 an/mit Orthesen (10) unterer Gliedmaßen, insbesondere Fuß-Orthesen, Knöchel-Fuß-Orthesen, Knie-Knöchel-Orthesen, Knie-Knöchel-Fuß-Orthesen, Hüfte-Knie-Knöchel-Orthesen, Hüfte-Knie-Orthesen, Hüfte-Knie-Knöchel-Fuß-Orthesen, Reziproke-Geh-Orthesen, Rumpf-Orthesen, Ellbogen-Orthesen, Ellbogen-Schulter-Orthesen, Rumpf-Schulter-Orthesen, Rumpf-Schulter-Ellbogen-Orthese, Schulter-Orthesen, Prothese mit Oberschenkelhülsen oder Prothesen mit Beckenkorb.

## Claims

1. Articulation for an orthosis having an upper part (12) and a foot stirrup (14), which are connected to one another via a swivel joint (16) with an axis of rotation so as to be pivotable relative to one another, whereby it is possible for the foot stirrup (14) to be acted upon dorsally and/or plantarly on both sides of the swivel joint (16) by a spring-elastic functional element (20, 20', 20", 20'", 20"") accommodated in/from a receiving element (18, 18'), **characterized in that** the receiving element (18, 18') can be detachably fastened to the upper part (12) via an attachment section (46) of the receiving element (18, 18') extending from a dorsal side (22) to a plantar side (24), and vice versa, transversely to a longitudinal axis (26) of the upper part (12) and comprises at least one, in particular two, receiving section(s) (50, 50') arranged on the attachment section (46) for receiving the functional element(s) (20, 20', 20", 20'", 20"") laterally of the upper part (12).

2. Articulation for an orthosis according to claim 1, **characterized in that** the attachment section (46) of the receiving element (18, 18') extends from the dorsal side (22) to the plantar side (24), and vice versa, substantially perpendicularly or perpendicularly or at an angle obliquely to the longitudinal axis (26) of the upper part (12).

3. Articulation for an orthosis according to claim 1 or 2, **characterized in that** the receiving element (18, 18') is formed in one piece with the attachment section (46) and the at least one, in particular the two, receiving section(s) (50, 50') arranged on the attachment section (46).

4. Articulation for an orthosis according to one of claims 1 to 3, **characterized in that** the upper part (12), to which the receiving element (18, 18') is detachably fastened, is formed in one piece.

5. Articulation for an orthosis according to one of claims 1 to 4, **characterized in that** the receiving element (18, 18') can be fastened to the upper part (12) in a form-fitting and/or force-fitting manner.

6. Articulation for an orthosis according to one of claims 1 to 5, **characterized in that** the upper part (12) is provided with a groove (36) or similar recess extending transversely, in particular substantially perpendicularly or perpendicularly or at an angle obliquely, to the longitudinal axis (26) of the upper part (12), into which the receiving element (18, 18'), in particular the attachment section (46) of the receiving element (18, 18'), can be inserted, preferably without play.

7. Articulation for an orthosis according to one of claims 1 to 6, **characterized in that** the receiving element (18, 18') can be fastened to the upper part (12) via a screw, latch, snap, or similar connection (48).

8. Articulation for an orthosis according to one of claims 1 to 7, **characterized in that** the at least one, in particular the two, receiving section(s) (50) of the receiving element (18, 18') formed on the attachment section (46) is/are arranged parallel to and/or at an angle oblique to the longitudinal axis (26) of the upper part (12) and/or symmetrically on both sides of the longitudinal axis (26) of the upper part (12) and with respect to each other.

9. Articulation for an orthosis according to one of claims 1 to 8, **characterized in that** the at least one, in particular the two, receiving section(s) (50) of the receiving element (18, 18') formed on the attachment section (46) laterally abut or contact the dorsal or front side or side edge (22), respectively, and/or the plantar or rear side or side edge (24), respectively, of the upper part (12).

10. Articulation for an orthosis according to one of claims 1 to 9, **characterized in that** the at least one, in particular the two, receiving section(s) (50) of the receiving element (18, 18') formed on the attachment section (46) is/are substantially encapsulated.

11. Articulation for an orthosis according to one of claims 1 to 10, **characterized in that** the at least one, in particular the two, receiving section(s) (50) formed on the attachment section (46) is/are each provided with an elongate recess (52) or bore for receiving the functional element (20, 20', 20", 20'", 20"").

12. Articulation for an orthosis according to one of claims 1 to 11, **characterized in that** the at least one, in particular the two, receiving section(s) (50, 50') formed on the attachment section (46) comprise(s) an opening (54), which can be closed, in particular by means of a screw connection (56), for insertion of the functional element (20, 20', 20", 20'", 20"").

13. Articulation for an orthosis according to one of claims 1 to 12, **characterized in that** the at least one, in particular the two, receiving section(s) (50) formed on the attachment section (46) comprise(s) an actuating element (58), which is axially displaceable in the receiving section (50) and is acted upon by the functional element (20, 20', 20", 20'", 20""), for dorsally and/or plantarly acting upon the foot stirrup (14).

14. Articulation for an orthosis according to one of claims 1 to 13, **characterized in that** the at least one, in particular the two, receiving section(s) (50) formed on the attachment section (46) comprise(s) an actuating element (58), acted upon by the functional element (20, 20', 20", 20'", 20"") and extending partially through a through bore (60) or similar passage, for dorsally and/or plantarly acting upon the foot stirrup (14).

15. Articulation for an orthosis according to one of claims 1 to 14, **characterized in that** the actuating element (58) for dorsally and/or plantarly acting upon the foot stirrup (14) is acted upon by a functional element (20, 20', 20", 20'", 20"") and is formed with a pin (62) or bolt extending through the through bore (60) or similar passage.

16. Articulation for an orthosis according to one of claims 1 to 15, **characterized in that** the at least one, in particular the two, receiving section(s) (50') formed on the attachment section (46) is/are substantially exposed.

17. Articulation for an orthosis according to one of claims 1 to 16, **characterized in that** the at least one, in particular the two, receiving section(s) (50') formed on the attachment section (46) is/are provided with screws (92), projections, or groove-shaped recesses for receiving and holding the functional element (20, 20', 20", 20'", 20"").

18. Articulation for an orthosis according to one of claims 1 to 17, **characterized in that** a wedge element (66), via which the freedom of movement of the articulation (10, 10', 10") for an orthosis is adjustable by means of at least one, preferably two, wedge surface(s) (68, 68'), is associated with the upper part (12).

19. Articulation for an orthosis according to claim 18, **characterized in that** the upper part (12) is provided with a groove (70) or similar recess extending substantially perpendicularly to the longitudinal axis (26) of the upper part (12), into which the wedge element (66) can be inserted and is detachably fastened, directly or indirectly via a carrier element (72).

20. Articulation for an orthosis according to one of claims 1 to 19, **characterized in that** the foot stirrup (14) comprises, on both sides of the swivel joint (16), a dorsal and a plantar projection (89, 89'; 90, 90') or similar projection, which can be acted upon by the spring-elastic functional element (20, 20', 20", 20'", 20"") accommodated in/from a receiving element (18, 18').

21. Articulation for an orthosis according to one of claims 18 to 20, **characterized in that** the foot stirrup (14) is assigned an actuator (82, 82', 82"), over which the functional position of the articulation (10, 10', 10") for an orthosis is adjustable by means of a stop area (84) that faces the at least one wedge surface (68, 68') and interacts with the at least one wedge surface (68, 68'), whereby the dorsal and the plantar projection (89, 89'; 90, 90') or similar projection of the foot stirrup (14) on both sides of the swivel joint (16) being integrally formed on the adjusting element (82, 82', 82").

22. Articulation for an orthosis according to one of claims 1 to 21, **characterized in that** the functional element(s) (20, 20', 20", 20'", 20"") is/are in the form of at least one spring, in particular compression spring, preferably disc spring (20, 20', 20") and/or helical spring (20'", 20"") and/or synthetic material element, or in particular tension spring, preferably helical spring and/or annular spring (96) made of spring-elastic material, and/or a gas compression spring and/or a gas tension spring, and/or a damper and/or a motor, in particular stepping motor, and/or a magnetic device, in particular a magnet, and/or a combination thereof and/or a package formed therefrom.

23. Modular system for forming an articulation for a orthosis according to one of claims 1 to 22, comprising an upper part (12), a foot stirrup (14), which are connected to one another via a swivel joint (16) with an axis of rotation so as to be pivotable relative to one another, a receiving element (18, 18'), and a spring-elastic functional element (20, 20', 20", 20'", 20"") which can be received in/from the receiving element (18, 18'), whereby the receiving element (18, 18') can be detachably fastened to the upper part (12) via an attachment section (46) of the receiving element (18, 18') extending from a dorsal side (22) to a plantar side (24), and vice versa, transversely to a longitudinal axis (26) of the upper part (12) and comprises at least one, in particular two, receiving section(s) (50, 50') arranged on the attachment section (46) for receiving the functional element(s) (20, 20', 20", 20'", 20"") laterally of the upper part (12).

24. Use of an articulation for an orthosis according to one of claims 1 to 23 on/with lower limb orthoses (10), in particular foot orthoses, ankle-foot orthoses, knee-ankle orthoses, knee-ankle-foot orthoses, hip-knee-ankle orthoses, hip-knee orthoses, hip-knee-ankle-foot orthoses, reciprocal walk orthoses, trunk orthoses, elbow orthoses, elbow-shoulder orthoses, trunk-shoulder orthoses, trunk-shoulder-elbow orthoses, shoulder orthoses, prosthesis with thigh sleeves or prosthesis with pelvic basket.

## Revendications

1. Articulation orthétique comprenant une partie supérieure (12) et un étrier de pied (14), qui sont reliés l'un à l'autre de manière à pouvoir pivoter l'un par rapport à l'autre par une articulation tournante (16) ayant un axe de rotation, l'étrier de pied (14) pouvant être soumis à une sollicitation dorsale et/ou plantaire, des deux côtés de l'articulation tournante (16), par un élément fonctionnel (20, 20', 20", 20'", 20"") présentant l'élasticité d'un ressort et reçu dans/par un élément de réception (18, 18'), **caractérisée en ce que** l'élément de réception (18, 18') est fixé de façon détachable à la partie supérieure (12) par une portion de fixation (46) de l'élément de réception (18, 18'), s'étendant d'un côté dorsal (22) vers un côté plantaire (24), et inversement, transversalement à un axe longitudinal (26) de la partie supérieure (12), et comprend au moins une portion de réception (50, 50'), en particulier deux, disposée(s) sur la portion de fixation (46) pour recevoir le/les élément(s) fonctionnel(s) (20, 20', 20", 20'", 20"") latéralement à côté de la partie supérieure (12).

2. Articulation orthétique selon la revendication 1, **caractérisée en ce que** la portion de fixation (46) de l'élément de réception (18, 18') s'étend du côté dorsal (22) vers le côté plantaire (24), et inversement, sensiblement perpendiculairement ou perpendiculairement ou selon un angle en oblique par rapport à l'axe longitudinal (26) de la partie supérieure (12).

3. Articulation orthétique selon la revendication 1 ou 2, **caractérisée en ce que** l'élément de réception (18, 18') est réalisé d'un seul tenant avec la portion de fixation (46) et avec ladite au moins une portion de réception (50, 50'), en particulier les deux, disposée(s) sur la portion de fixation (46).

4. Articulation orthétique selon l'une des revendications 1 à 3, **caractérisée en ce que** la partie supérieure (12), à laquelle l'élément de réception (18, 18') est fixé de manière détachable, est réalisée d'un seul tenant.

5. Articulation orthétique selon l'une des revendications 1 à 4, **caractérisée en ce que** l'élément de réception (18, 18') peut être fixé à la partie supérieure (12) par coopération de forme et/ou de force.

6. Articulation orthétique selon l'une des revendications 1 à 5, **caractérisée en ce que** la partie supérieure (12) est pourvue d'une rainure (36) ou d'une échancrure similaire s'étendant transversalement, en particulier sensiblement perpendiculairement ou perpendiculairement ou selon un angle en oblique par rapport à l'axe longitudinal (26) de la partie supérieure (12), dans laquelle l'élément de réception (18, 18'), en particulier la portion de fixation (46) de l'élément de réception (18, 18'), peut être inséré(e), de préférence sans jeu.

7. Articulation orthétique selon l'une des revendications 1 à 6, **caractérisée en ce que** l'élément de réception (18, 18') peut être fixé à la partie supérieure (12) par une liaison par vis, par enclenchement, par encliquetage ou similaire (48) .

8. Articulation orthétique selon l'une des revendications 1 à 7, **caractérisée en ce que** ladite au moins une portion de réception (50) de l'élément de réception (18, 18'), en particulier les deux, conformée(s) sur la portion de fixation (46), est/sont disposée(s) parallèlement et/ou selon un angle en oblique par rapport à l'axe longitudinal (26) de la partie supérieure (12) et/ou symétriquement de part et d'autre de l'axe longitudinal (26) de la partie supérieure (12) et l'une par rapport à l'autre.

9. Articulation orthétique selon l'une des revendications 1 à 8, **caractérisée en ce que** ladite au moins une portion de réception (50) de l'élément de réception (18, 18'), en particulier les deux, conformée(s) sur la portion de fixation (46) s'appuie(nt) contre ou touche(nt) latéralement le/les côté(s) ou bord(s) latéral/latéraux (22) dorsal/dorsaux ou antérieur(s) et/ou le/les côté(s) ou bord(s) latéral/latéraux (24) plantaire(s) ou postérieur(s) de la partie supérieure (12).

10. Articulation orthétique selon l'une des revendications 1 à 9, **caractérisée en ce que** ladite au moins une portion de réception (50) de l'élément de réception (18, 18'), en particulier les deux, conformée(s) sur la portion de fixation (46) est/sont sensiblement encapsulée(s).

11. Articulation orthétique selon l'une des revendications 1 à 10, **caractérisée en ce que** ladite au moins une portion de réception (50), en particulier les deux, conformée(s) sur la portion de fixation (46) est/sont pourvue(s) chacune d'une échancrure (52) ou d'un perçage allongé(e) pour recevoir l'élément fonctionnel (20, 20', 20", 20'", 20"").

12. Articulation orthétique selon l'une des revendications 1 à 11, **caractérisée en ce que** ladite au moins une portion de réception (50, 50'), en particulier les deux, conformée(s) sur la portion de fixation (46) présente(nt) une ouverture (54) pouvant être refermée, en particulier au moyen d'un raccord vissé (56), pour insérer l'élément fonctionnel (20, 20', 20", 20'", 20"").

13. Articulation orthétique selon l'une des revendications 1 à 12, **caractérisée en ce que** ladite au moins une portion de réception (50), en particulier les deux, conformée(s) sur la portion de fixation (46) présente(nt) un élément d'actionnement (58) déplaçable axialement dans la portion de réception (50) et sollicité par l'élément fonctionnel (20, 20', 20", 20'", 20""), pour la sollicitation dorsale et/ou plantaire de l'étrier de pied (14).

14. Articulation orthétique selon l'une des revendications 1 à 13, **caractérisée en ce que** ladite au moins une portion de réception (50), en particulier les deux, conformée(s) sur la portion de fixation (46) présente(nt) un élément d'actionnement (58) sollicité par l'élément fonctionnel (20, 20', 20", 20'", 20"") et s'étendant partiellement à travers un perçage traversant (60) ou un passage similaire, pour la sollicitation dorsale et/ou plantaire de l'étrier de pied (14).

15. Articulation orthétique selon l'une des revendications 1 à 14, **caractérisée en ce que** l'élément d'actionnement (58) pour la sollicitation dorsale et/ou plantaire de l'étrier de pied (14) est sollicité par un élément fonctionnel (20, 20', 20", 20'", 20"") et est formé avec une tige (62) ou un goujon s'étendant à travers le perçage traversant (60) ou le passage similaire.

16. Articulation orthétique selon l'une des revendications 1 à 15, **caractérisée en ce que** ladite au moins une portion de réception (50'), en particulier les deux, conformée(s) sur la portion de fixation (46) est/sont réalisée (s) de façon sensiblement dégagée.

17. Articulation orthétique selon l'une des revendications 1 à 16, **caractérisée en ce que** ladite au moins une portion de réception (50'), en particulier les deux, conformée(s) sur la portion de fixation (46) est/sont pourvue(s) de vis (92), de saillies ou d'échancrures en forme de rainures pour recevoir et maintenir l'élément fonctionnel (20, 20', 20", 20'", 20"").

18. Articulation orthétique selon l'une des revendications 1 à 17, **caractérisée en ce que** la partie supérieure (12) est associée à un élément en forme de coin (66) qui permet de régler la liberté de mouvement de l'articulation orthétique (10, 10', 10") au moyen d'au moins une, de préférence deux, surface(s) en forme de coin (68, 68').

19. Articulation orthétique selon la revendication 18, **caractérisée en ce que** la partie supérieure (12) est pourvue d'une rainure (70) ou d'une échancrure similaire s'étendant sensiblement perpendiculairement à l'axe longitudinal (26) de la partie supérieure (12), dans laquelle l'élément en forme de coin (66) peut être inséré et fixé de manière détachable, directement ou indirectement par le biais d'un élément de support (72).

20. Articulation orthétique selon l'une des revendications 1 à 19, **caractérisée en ce que** l'étrier de pied (14) présente des saillies dorsale et plantaire (89, 89'; 90, 90') ou des porte-à-faux similaires de part et d'autre de l'articulation tournante (16), qui peuvent être sollicité(e)s par l'élément fonctionnel (20, 20', 20", 20'", 20"") présentant l'élasticité d'un ressort et reçu dans/par un élément de réception (18, 18').

21. Articulation orthétique selon l'une des revendications 18 à 20, **caractérisée en ce que** l'étrier de pied (14) est associé à un élément de réglage (82, 82', 82") par lequel la position fonctionnelle de l'articulation orthétique (10, 10', 10") est réglable au moyen d'une surface de butée (84) tournée vers ladite au moins une surface en coin (68, 68') et coopérant avec ladite au moins une surface en coin (68, 68'), les saillies dorsale et plantaire (89, 89'; 90, 90') ou les porte-à-faux similaires de l'étrier de pied (14) de part et d'autre de l'articulation tournante (16) étant conformé(e)s d'un seul tenant sur l'élément de réglage (82, 82', 82").

22. Articulation orthétique selon l'une des revendications 1 à 21, **caractérisée en ce que** le/les élément(s) fonctionnel(s) (20, 20', 20'', 20''', 20"") est/sont réalisé(s) comme au moins un ressort, en particulier un ressort de compression, de préférence une rondelle-ressort (20, 20', 20") et/ou un ressort hélicoïdal (20'", 20"") et/ou un élément en matière plastique, ou en particulier comme un ressort de traction, de préférence un ressort hélicoïdal et/ou un ressort annulaire (96) en matériau élastique, et/ou comme un ressort de compression à gaz et/ou un ressort de traction à gaz, et/ou un amortisseur et/ou un moteur, en particulier un moteur pas à pas, et/ou comme un dispositif magnétique, en particulier un aimant, et/ou une combinaison de ceux-ci et/ou un paquet formé à partir de ceux-ci.

23. Système modulaire pour former une articulation orthétique selon l'une des revendications 1 à 22, comprenant une partie supérieure (12) et un étrier de pied (14), qui sont reliés l'un à l'autre de manière à pouvoir pivoter l'un par rapport à l'autre par une articulation tournante (16) ayant un axe de rotation, un élément de réception (18, 18') et un élément fonctionnel (20, 20', 20", 20'", 20"") présentant l'élasticité d'un ressort et pouvant être reçu dans/par l'élément de réception (18, 18'), dans lequel l'élément de réception (18, 18') peut être fixé de façon détachable à la partie supérieure (12) par une portion de fixation (46) de l'élément de réception (18, 18'), s'étendant d'un côté dorsal (22) vers un côté plantaire (24), et inversement, transversalement à un axe longitudinal (26) de la partie supérieure (12), et comprend au moins une portion de réception (50, 50'), en particulier deux, disposée(s) sur la portion de fixation (46) pour recevoir le/les élément(s) fonctionnel(s) (20, 20', 20", 20'", 20"") latéralement à côté de la partie supérieure (12).

24. Utilisation d'une articulation orthétique selon l'une des revendications 1 à 23 sur/avec des orthèses (10) de membres inférieurs, en particulier des orthèses de pied, des orthèses de cheville-pied, des orthèses de genou-cheville, des orthèses de genou-cheville-pied, des orthèses de hanche-genou-cheville, des orthèses de hanche-genou, des orthèses de hanche-genou-cheville-pied, des orthèses de marche réciproque, des orthèses de tronc, des orthèses de coude, des orthèses de coude-épaule, des orthèses de tronc-épaule, des orthèses de tronc-épaule-coude, des orthèses d'épaule, des prothèses avec douille de fémur ou des prothèses avec attelle pelvienne.
